Europäisches Patentamt

European Patent Office

Office européen des brevets

(11) Publication number: **0 192 675**
**B1**

(12) **EUROPEAN PATENT SPECIFICATION**

(45) Date of publication of patent specification: **19.09.90**

(21) Application number: **85904112.1**

(22) Date of filing: **21.08.85**

(86) International application number:
**PCT/EP85/00428**

(87) International publication number:
**WO 86/01411 13.03.86 Gazette 86/06**

(51) Int. Cl.[5]: **A 61 K 39/395, C 12 N 9/72,
G 01 N 33/577, C 12 P 21/00**

(54) **ANTI-UROKINASE MONOCLONAL ANTIBODY; ITS PRODUCTION AND USE.**

(30) Priority: **31.08.84 GB 8422007
29.03.85 GB 8508244
26.04.85 GB 8510716**

(43) Date of publication of application:
**03.09.86 Bulletin 86/36**

(45) Publication of the grant of the patent:
**19.09.90 Bulletin 90/38**

(84) Designated Contracting States:
**AT BE CH DE FR GB IT LI LU NL SE**

(56) References cited:
**EP-A-0 084 344
WO-A-85/00663
BE-A-89 625 3
CA-A-1 028 619
Biochemistry, vol. 21, no. 25, 1982, L.S. Nielsen et al.:"Purification of zymogen to plasminogen activator from human glioblastoma cells by affinity chromatography with monoclonal antibody", pp. 6410-6415
Proceedings of the National Academy of Sciences USA, vol. 81, Jan. 1984, Salerno et al.:"Monoclonal antibodies to human urokinase identify the single-chair pro-urokinase precursor", pp. 110-114**

(73) Proprietor: **GRUPPO LEPETIT S.P.A.
23, Via G. Murat
I-20159 Milano (IT)**

(72) Inventor: **NOLLI, Maria, Luisa
15, Via Cavallini
I-27100 Pavia (IT)**
Inventor: **CORTI, Angelo
2, Via Mozzi
I-24100 Bergamo (IT)**
Inventor: **SOFFIENTINI, Adolfo
168, Via Fratelli di Dio
I-20099 Sesto San Giovanni (MI) (IT)**
Inventor: **CASSANI, Giovanni
3, Via Vittadini
I-27100 Pavia (IT)**
Inventor: **PARENTI, Francesco
24, Via Benvenuto Cellini
I-20020 Lainate (MI) (IT)**

(56) References cited:
**Patent Abstracts of Japan, vol. 8, no. 126(C-228)(1563), 13 June 1984 & JP, A, 57146822 (ASAHI KASEI KOGYO K.K.) 28 February 1984**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a class of new IgG₁ monoclonal antibodies, directed to an epitope on human urokinase, which selectively binds to high molecular weight urokinase (54,000), or a single-chain precursor thereof, without binding to low molecular weight urokinase (33,000), has an affinity constant for urokinase which is suitable for use in an immunoadsorption-based purification system and does not impair the enzymatic activity of urokinase.

The monoclonal antibodies of the invention, when coupled to a suitable matrix, give an immunoadsorbent matrix capable of purifying human urokinase or a single chain precursor thereof from biological sources. These immunoadsorbents, because of the optimal affinity of the antibody for its antigen, are particularly suitable for purifying human urokinase or a single-chain precursor thereof also on industrial scale.

The monoclonal antibodies of the invention are also suitable for use in analytical methods for detecting high molecular weight human urokinase (HMW urokinase) or a single chain precursor thereof such as immunofluorescence-based assays, radioimmunoassays and enzyme-immunoassays.

Human urokinase is a known plasminogen activator (PA) which is mainly produced by kidney cells. Its major physiological role seems to be that of an ativator of the fibrinolytic system. Urokinase is therefore indicated for the treatment of acute vascular diseases such as myocardial infarct, stroke, pulmonary embolism, deep vein thrombosis, peripheral arterial occlusion, hemorrhage and retinal vein occlusion.

Plasminogen activators (PA) are serine proteases which convert plasminogen into plasmin, a protease with high specificity for fibrin which is the main constituent of blood clots. At least two immunologically distinct types of PAs have been isolated from cultured malignant cell lines: those related to the tissue plasminogen activator (TPA) (see Rijken D. C., Wijngaard G. Zaal-de Jong M., Welbergen J. (1079) *Biochim. Biophys Acta* 380, 140) and those related to the urinary activator (UPA) (also called urokinase) (see Williams J. R. B. (1951) *Br. J. Exp. Pathol.* 32, 530). The urokinase type, originally purified as a two chain protein from urine, has been recently isolated in a proenzyme form (called pro-urokinase), for instance, from a human epidermoid carcinoma cell line HEp3 (see Wun T. D., Ossowsky L. and Reich E. (198) *J. Biol. Chem.* 257, 7262), from human glioblastoma cells (see Nielsen L. S. *et al.* (1982) *Biochem* 21, 6410), from urine (see Husain S. C. et al. (1983) *Arch. Biochem. Biophys* 220, 31) and from the human kidney permanent cell line TCL-598 (see Kohno T. *et al.* (1984) *Biotechnol* 2, 628). A proenzyme immunologically related to urokinase has been recently identified also from the supernatant of the human epidermoid carcinoma cell line A431.

This pro-urokinase zymogen is single chain protein of 50,000—54,000 with no or low amidolytic activity and high fibrin affinity, it is resistant to diisopropylfluorophosphate treatment and inactivation by plasma inhibitors (see Gurewich *et al.* (1984) *J. Clin Invest.* 73, 1731) and it is convertible into the two-chain active enzyme (urokinase) by treatment with plasmin.

The human urokinase molecule has been isolated in several biologically active forms, but it mainly consists of a high molecular weight form (MW about 54,000) and a low molecular weight form (MW about 33,000). The low molecular weight for (LMW) is derived from the high molecular weight form (HMW) by enzymatic cleavage.

The HMW form consists of a 30,000 heavy chain (B-chain) and a 20,000 light chain (A-chain) linked by a disulfide bond. The LMW form consists of the whole B-chain which contains the active site and a small proteolytically resistant fragment (MW = 2,427) derived by the A-chain linked by a disulfide bond. The A-chain is proteolytically splitted into a 18,000 fragment and the above 2,427 fragment. It seems that the LMW urokinase is less active than the HMW urokinase in accelerating cot lysis *in vivo* (M. Samama et al., Thromb. Haemostas. *40*, 578—580, (1979) and G. Murano et al., Blood, *55*, 430—436 (1980)). Human urokinase is at present mainly obtained from biological sources such as human urine, tissue cultures, particularly normal and tumoral kidney cell cultures, by means of conventional purification techniques. Human urokinase zymogen may play a similar physiological role, once activated to urokinase. Recently it was reported that human urokinase was producd by DNA technology (see Eur. Pat. Appln. Publ. No. 92182).

Monoclonal antibodies against urokinase have been described since the pioneer work on monoclonal antibody-producing cell-hybrids of C. Milstein and G. Köhler described in Nature, *256*, 495—497 (1975). For instance, Belgian Patent n. 896,253 discloses several monoclonal antibodies obtained by immunizing mice with a low molecular weight urokinase (33,000). These monoclonal antibodies are evidently directed against an epitope on the low molecular weight form of urokinase. One of these antibodies, denominated AAU2, was selected and used in the purification of urokinase (see P. Herion and A. Bollen, Bioscience Reports *3*, 373—379 (1983)). The obtained urokinase, of course, should contain both low and high molecular weight forms. D. Vetterlein and G. J. Colton described the purification of urokinase by means of immobilized monoclonal antibodies against the urokinase light chain (Thromb. Haemostas (Stuttgart) *49*, 24—27 (1983)). The described purification factor is less than 2.4 (from 60,000—70,000 CTA units/mg to 142,000—167,000 CTA units/mg; see page 25 right column, lines 10—11), the urokinase bond to the immunoadsorbent in the described experiment with urine (300 ml urine per 1 ml of resin; see page 26, second paragraph of the notes to fig. 2) is not suitable for industrial scale purification. Moreover, these authors pointed out that the problem of industrial purification of urokinase using a monoclonal antibody immunoadsorbent is still to be solved. ("However, large-scale uses of this affinity technique must still be demonstrated", page 27, left column, lines 13—14).

European Patent Application publication number 84344 discloses some anti-urokinase monoclonal antibodies in broad terms. It states that they are useful for purifying urokinase and for diagnostic and analytical purposes. However, the final product, urokinase, is neither characterized for its content in the high or low molecular weight form, nor is it characterized for its content in biologically active or toxic contaminants. G. Salerno et al., Proc. Natl. Acad. Sci. USA, *81*, pages 110—114 (1984) describes other anti-urokinase monoclonal antibodies which, as shown below, bind both the high and the low molecular weight forms of urokinase and possess an affinity constant which is not suitable for an efficient affinity chromatography. Other immunoadsorbents bearing "anti-urokinase" monoclonal antibodies were described by L. S. Nielsen et al., Biochemistry, *21*, 6410—6415 (1982) and K. Kaltoft et al., Proc. Natl. Acad. Sci. USA, *79*, 3720—3723 (1982). The described monoclonal antibodies inhibit the enzymatic activity of a plasminogen activator which they denominate HPA 52. However, the amount of antibody linked to the matrix per milliliter of matrix and the capacities of the final modified matrix are unsuitable for industrial scale use. Moreover, the use of two sequential chromatographic-steps are necessary to purify HPA 52 from glioblastoma cell culture medium (see Biochemistry 21, 1982, Table I, page 6412).

The present invention provides an anti-human urokinase monoclonal antibody having the following characteristics:

a) it is an IgG$_1$

b) it has an affinity constant of $(1.42 \pm 1.5) \times 10^7$ l mole$^{-1}$ for immobilized urokinase, when measured essentially following the procedure described by Tsapis et al. in Eur. J. Biochem. *64*, 369 (1976)

c) it binds to high molecular weight (54,000) urokinase or a single chain precursor thereof without binding the low molecular weight (33,000) urokinase

d) it binds the 18,000 proteolytic fragment of the A-chain

e) it does not cross-react with serum or urine enzymatically active endopeptidases other than urokinase.

In view of the characteristic feature of the monoclonal antibodies of the invention of binding both high molecular weight urokinase and the urokinase precursor, in the following description and claims when dealing with the specificity and binding properties of the monoclonal antibodies of the invention, the term "urokinase" encompasses both the high molecular weight form of urokinase and the urokinase precursors. The reference, in the same context, to the "high molecular form of urokinase" is to be understood as encompassing also its precursors which are, similarly, specifically bound by a monoclonal antibody of the invention. In the present disclosure and claim the term "urokinase precursor" refers to "natural" single chain precursors of urokinase like the above mentioned urokinase zymogen as well as to urokinase precursors or related substances obtained by rec-DNA techniques which have the common features of binding a monoclonal antibody of the invention.

An anti-urokinase monoclonal antibody of the invention is obtained by somatic cell fusion of mouse myeloma cells of the "non-secreting" type, i.e. myeloma cells which do not secrete immunoglobulins, with spleen cells of mice previously immunized with urokinase. Briefly, Balb/c mice are immunized with highly purified 54,000 urokinase and the removed spleen cells are fused with non-secreting myeloma cells which possess certain genetic defects which make them uncapable of surviving in a certain medium which becomes very useful for the subsequent isolation of hybrids cells. An example of such cell line is that known as X63-Ag8653 which was described by Kearney et al., in J. Immunol. *123*, 1548—1550 (1979) and which is known and available to the man skilled in the art.

Such myeloma cell lines are generally available to the public through many institutions of the scientific community such as The Salk Institute, Cell Distribution Center, P.O. Box 1809, San Diego, California 92112, and Institute for Medical Research, NIGMS Cell Repository, Copewood and Davis Streets, Camden, New Jersey 08103.

The cell fusion is conducted in the presence of polyethylene glycol, preferably polyethylene glycol (PEG) 6000.

Highly purified 54,000 urokinase (HMW urokinase) is commercially available from many sources. A suitable highly purified urokinase preparation has a high titer in International Units (IU), a high ratio fibrinolytic/esterolytic activity and very low amounts or a practical absence of thromboplastinic contaminants. A preferred highly purified urokinase preparation is that commercialized under the trade name PERSOLV® (Gruppo Lepetit S.p.A.) which has a fibrinolytic activity higher than 100,000 IU/mg, a ratio fibrinolytic/esterolytic activity higher than 1.4, is apyretic in rabbits at doses higher than 20,000 IU/Kg and has a zero-coagulant activity at plasma concentrations as high as 200 IU/ml.

The immunization schedule is preferably characterized by a low dose administratin of urokinase. A typical immunization schedule in mice includes a first administration of a low dose of highly purified 54,000 MW urokinase (120,000 IU/mg; about 10 μg) in complete Freund adjuvant 60 days before fusion, i.p.; a second i.p. injection of the same amount in incomplete Freund adjuvant 30 days before fusion and a final i.v. booster of the same amount 5 days before fusion.

Only those animals which 10 days before fusion have an anti-urokinase antibody titer higher than 1:10,000 are given the final booster and further processed. Spleens are removed and the spleen cells are fused with the myeloma cells at a ratio of about $10^8$ to $5 \times 10^7$ cells. After fusion, the cells are cultured in a suitable medium such as Dulbecco's modified Eagle medium containing about 10% fetal calf serum and the fused cells are selected on a Hypoxantine, Aminopterine, Thymidine medium (HAT). The supernatants of the cell cultures are assayed for anti-urokinase antibodies by means of ELISA methods involving anti-urokinase "conventional" antiserum. The purification of the thus identified anti-urokinase monolonal antibodies can be conducted by mean of ion-exchange chromatography or by affinity chromatography on a urokinase-agarose column, while gel electrophoresis is a convenient means for evaluating the homogeneity of the obtained product. These preparations of anti-urokinase monoclonal antibodies are then evaluated for class specificity and affinity constant for the antigen immobilized on agarose. The class specificity is evaluated according to the Ouchterlony's double immunodiffusion technique (Acta Pathol. Microbiol. Scand. 26, 507, (1949)); the affinity constant is determined by equilibrium binding experiments conducted essentially following the method of Tsapis et al. (Tsapis A., Rogard N., Alfsen A., and Nihaesco C. (1976) Eur. J. Biochem. 64, 369). In these experiments increasing concentrations of essentially pure monoclonal antibody previously dialyzed in phosphate buffered saline pH 7.2 are added to an urokiinase bearing matrix, such as a urokinase-agarose matrix. The concentration of the unbound antibody is determined spectrophotometrically, the concentration of bound antibody is calculated by difference from the total antibody added, and the binding data are elaborated according to the known statistical methods such as the method described by G. Scatchard in Ann. N.Y. Acad. Sci. 51, 660—672 (1949).

The monoclonal antibody of the present invention has an affinity constant of $(1.42 \pm 1.5) \times 10^7$ 1 mole$^{-1}$ in the above described assay.

An object of the present invention is the use of the monoclonal antibodies of the invention in an immunoadsorption system for the purification of urokinase on industrial scale.

It will be appreciated by those skilled in the art that not any monoclonal antibody directed against a certain antigen (urokinase, in the present case) is suitable for use in an industrial scale process. It is known in fact that there is usually a gap between "lab" scale and "industrial" scale operations. The change in magnitude in fact considerably amplifies the problems to be solved and, in many instances, a solution which is satisfactory for a lab-scale operation is unsuitable for an industrial scale operation.

In the present case, the suitable monoclonal antibody should have an optimal affinity for the antigen in order to efficiently bind the antigen at such conditions that it is possible to rinse the bound antigen without significantly releasing it and to release it from the matrix at significantly different conditions.

The releasing conditions, in turn, should be mild enough to avoid any damage either to the antigen or to the immunoadsorbent.

Since the affinity of the antibody for the antigen controls, to a certain extent, the capacity of the final immunoadsorbent, a low affinity of the antibody is generally related to a too low capacity of the immunoadsorbent with consequent low yields of purification (e.g. because of an activity loss during rinsing); on the contrary, a high affinity of the antibody is generally related to a too strong binding of the antigen to the immunoadsorbent with consequent low yields because of e.g. difficulties of elution or too drastic (and possibly denaturating elution conditions). However, the affinity constant of the monoclonal antibody and the capacity of the corresponding immunoadsorbent are not the sole critical parameters in an industrial scale purification process based on immunoadsorption since also the selectivity of the monoclonal antibody plays a very important role in this scenario.

In fact, the monclonal antibodies of the invention bind the high molecular weight urokinase but do not bind the low molecular weight form or any other serum or urine enzymatically active forms of endopeptidase.

In particular they do not cross-react with the known serum or urine thromboplastinic contaminants. In addition, the monoclonal antibodies of the invention maintain this specificity also when linked to the matrix in the final immunoabsorbent. In particular, the monoclonal antibodies of the invention, which are characterized by above described specificity have, as already said, an affinity constant of $(1.42 \pm 1.5) \times 10^7$ mole$^{-1}$ in the above described assay, when coupled to the suitable matrix give an immunoadsorbent with a capacity higher than 150,000 IU/mg.

Monoclonal antibodies of the invention are therefore IgG$_1$ having an affinity constant of about $(1.42 \pm 1.5) \times 10^7$ I mole$^{-1}$ in the above described assay which uses immobilized urokinase, a good specificity for the high molecular weight form of urokinase or single chain precursor thereof without binding to the low molecular weight form or cross-reacting with serum or urine endoproteases other than urokinase, and, once linked to the suitable matrix, are capable of giving immunoadsorbent matrices with capacity higher than 150,000 IU/mg, are capable of binding the antigen at a pH between 4.5 and 8.0, even in the presence up to about 0.5 M aqueous sodium chloride, and releasing the bound antibody at a pH between 3.0 and 4.0 in 0.5—1 M aqueous sodium chloride. The preferred monoclonal antibody of the invention is denominated 5B4 and possesses the following characteristics:

a) it belong to IgG$_1$ class

b) it has an affinity constant of about $1.42 \times 10^7$ I mole$^{-1}$, when measured essentially following the procedure described by Tsapis et al. in Eur. J. Biochem. 64, 369 (1976)

c) it specifically binds the high molecular form (54,000) of urokinase or a precursor thereof without binding the low molecular (33,000) form or any serum or urine thromboplastinic contaminant of urokinase

d) it binds the 18,000 proteolytic fragment of the A-chain

e) it does not impaire the enzymatic activity of the desorbed urokinase

f) it has an isoelectric point of about 5.75

Representative matrices suitable for coupling with the monoclonal antibodies of the invention are cross-linked dextran and controlled-pore cross-linked dextrane, agarose, modified and activated agarose such as carboxymethyl agarose, N-hydroxysuccinimide ester derivatives of cross-linked agarose, cellulose and carboxymethyl cellulose. These matrices are in many instances commercially available either in activated or non-activated form. The activated matrices are generally preferred because of the known handling advantages in the coupling reaction.

The preferred matrix for the preparation of the immunoadsorbent of the invention is activated agarose.

The coupling of the monoclonal antibody of the invention to the matrix is obtainable by known per se techniques.

When the matrix used is a non-activated one, the coupling is preferably obtained by means of known activating agents such as cyanogen bromide, epichloridin, 1,4-bis-(2,3-diepoxypropoxy)butane and 3-aminopropyltriethoxysilane.

One of the preferred uses of the immunoadsorbent of the invention is in the purification of 54,000 MW urokinase or a precursor thereof from biologial sources, especially in industrial scale purifications. In addition, this immunoadsorbent can be usefully employed in analytial test systems such as radioimmunoasssay, enzyme-linked immunoassay and other known competitive binding assays, because of the affinity for the antigen and specificity of the linked monoclonal antibody.

A further object of the present invention is a process for purifying 54,000 MW urokinase (HMW urokinase) or a precursor thereof from biological sources which comprises contacting a biological source or a concentrate thereof with an immunoadsorbent of the invention at a pH between 4.5 and 8.0 in order to selectively bind the 54,000 MW urokinase to the immunoadsorbent of the invention, rinsing it with a buffered solution at a pH between 6 and 8 and releasing the bound antigen from the immunoadsorbent by eluting with an aqueous solution at a pH between 3.0 and 4.0, optionally containing 0.5—1 M aqueous sodium chloride.

In this process, the binding of the antigen to the immunoadsorbent may be obtained also in the presence of aqueous sodium chloride in concentrations up to 0.5 M, while the release of the antigen from the complex antigen/immunoadsorbent may be obtained in the presence of a concentration of aqueous sodium chloride from 0.5 to 1 M.

As it will be appreciated by those skilled in the art, the aqueous sodium chloride solution may be replaced by any aqueous solution of equivalent strength which does not unfavorably interfere with the course of the purification process.

In the present specification and claims the expression "biological sources" includes biological fluids such as urine and blood, tissue culture fluids and fermentation broths of genetically engineered microorganisms capable of producing urokinase or a plasminogen activator recognized by the monoclonal antibody of the invention.

As already said, the immunoadsorbent of the invention is capable of purifying the high molecular weight form of urokinase directly from biological sources; for instance, when the biological source is human urine a urokinase product is obtained which has the following characteristics:

a) it has a fibrinolytic titer higher than 130,000 IU/ml

b) it is rich in high molecular weight urokinase

c) it is practically devoid of low molecular weight urokinase

d) it has a ratio fibrinolytic/esterasic activity higher than 2,000

e) it is practically devoid of thromboplastinic contaminants (zero-coagulant activity at concentrations of about 200 IU/ml)

A urokinase product with characteristics which are substantially as above is obtained starting from any other biological source.

In some instances, in the practice of industrial scale purifications, it may be convenient to submit a concentrate of the biological fluid, instead of the biological fluid *as such*, to the purification by means of the immunoadsorbent of the invention in order to reduce the volumes of fluid to be contacted with the immunoadsorbent (or passed through a column containing the immunoadsorbent) and consequently reduce the time and the costs of the whole operation.

Also in this case the immunoadsorbent of the invention maintains its efficiency and gives a purified product which has the above described characteristics.

For better clarifying the problem of the choice of the monoclonal antibody suitable for use in the purification process which is one of the object of the present invention, the following table report the results of a comparison between the preferred monoclonal antibody of the invention, which is denominated 5B4 and a monoclonal antibody described by Salerno et al., Proc. Natl. Acad. Sci. USA, *81*, 110 (1984), which was denominated mAB 105.1F 10, which has an affinity constant similar to that of 5B4.

TABLE I

Affinity constants of monoclonal
antibody (mAB) 5B4 and 105. 1F 10

| MAB | Ka (l mole$^{-1}$) |
| --- | --- |
| 105.1F 10 | 4.92 x 10$^6$ |
| 5B4 | 1.42 x 10$^7$ |

These two monoclonal antibodies which belong to the same class (both are IgG$_1$) when linked in equal amounts to equal amounts of activated agarose (Affi-gel® 10) give substantially different results in the purification of preparations of urokinase.

More particularly, when preparations of the same purity of urokinase (897 esterolytic units) are passed through the two different immunoadsorbents, the bound fraction is less than 10% in the case of 105.1F 10 and higher than 70% (namely 77.5%) in the case of mAB 5B4.

The monoclonal antibodies of the invention because of their affinity and specificity for the antigen can be usefully employed in assay or detection procedures. They can be labelled with various markers such as fluorescent dyes, radioisotopes and enzymes, and used in immunofluorescence, radioimmunoassays or enzymeimmunoassays.

Another object of the present invention is therefore an enzyme-immunoassay, radioimmunoassay or immunofluorescence assay of human urokinase or a precursor thereof by means of monoclonal antibody of the invention. Human urokinase, is usually assayed by the fibrinolytic or the esterolytic methods. The first method is based on the clot lysis produced by the activation of plasminogen to plasmin by urokinase while the second one is based on the direct hydrolysis of the synthetic substrate acetyl-lysyl-methyl-ester (ALME) by urokinase. Therefore these assays detect the active form of the enzyme only. Inactive precursors of UK, such as preprourokinase or prourokinase or inhibitor-enzyme complexes escape detection. On the contrary, immunological assays, that are based on antigenic properties of the molecule and not on its activity, allow detection of active and inactive forms of the enzyme.

A very convenient immunoassay procedure which is useful for detecting or assaying urokinase or an "inactive" precursor thereof (such as a urokinase zymogen or pro-urokinase) is a double antibody sandwich procedure which comprises:

a) causing a test solution containing the antigen to react with a first anti-urokinase antibody immobilized on a solid phase carrier to bind the antigen to the solid phase,

b) contacting with the bound antigen a solution containing a second anti-urokinase antibody having linked thereto a determinable marker, wherein one of the said first and second antibodies is a monoclonal antibody of the invention,

c) detecting or assaying the antigen in accordance with the extent to which the determinable marker has been bound to the solid phase carrier.

The "first" antibody is preferably selected from an anti-urokinase antiserum, anti-urokinase purified immunoglobulin G (IgG$_1$) and a monoclonal antibody which is known to bind to a site of the antigen which is different from the binding site of the "second" antibody (and obviously not interfering with the binding of said "second" antibody). The "test solution" may be any solution thought to contain the antigen bound by the monoclonal antibody of the invention and includes biological fluids, fermentation broths e.g. of genetically engineered microorganisms and cell culture broths and extracts. The "second" antibody is preferably a monoclonal antibody of the invention linked to a suitable determinable ligand such as an enzyme, a fluorescent dye or a radioactive group. A preferred determinable marker is an enzyme and the preferred "marker" is horseradish peroxidase. The preferred monoclonal antibody of the invention is the 5B4 monoclonal antibody described above.

When both the "first" and the "second" antibody are monoclonal (one of which is a monoclonal antibody of the invention while the other is another anti-human urokinase monoclonal antibody which binds a site on the antigen which is different from the antibody of the invention and whose binding does not interfere with the binding of the monoclonal antibody of the invention) they are in general interchangeable in the above assay procedure. In other words one of the two monoclonal antibodies may the "first" and the other the "second" or vice versa. On the contrary, in the case of an assay wherein one of the "antibody" is represented by a conventional antiserum it will be almost exclusively employed as the "first" antibody, since, the efficiency of the system is higher than in the reverse situation.

The coupling of the monoclonal antibody with the enzyme marker is obtained according to known per se techniques. A preferred coupling reagent is glutaraldehyde. When the detectable marker is an enzyme, a color developing chromogenic enzyme substrate is employed which is a known synthetic substrate of the labelling enzyme which, after incubating with the enzyme, originates a coloured derivative whose intensity is detected and evaluated in comparison with the colour developed by standard preparations of the test

6

compound. In addition to these quantitative aspects, this method can in any case be used also for qualitative purposes, in order to detect simply the presence of the antigen in an unknown sample.

The solid phase carrier includes particles of cellulose, polyacrylamide, cross-linked dextrans, silicone rubber, microcrystalline glass and plastic and preferably preformed materials such as tubes, discs, or microplates moulded from plastic such as polystyrene, polypropylene and polyvinyl. Polyvinyl microplates are the preferred solid phase carrier. The preferred application range of the method of the invention is from 15 to 100 mg/ml of antigen. As it is evident to the man skilled in the art, when samples having an antigen concentration falling outside these limits have to be tested, they must be previously diluted or (if proper) concentrated in order to tentatively enter this optimal concentration range. The method is then validated by evaluating the inter- and intra-assay precision and the percentage of analytical recovery. Intra-assay precision (CV) is generally between 4 and 8%, inter-assay precision (CV) is generally between 7 and 23%, while the analytical recovery is between 87 and 114%. The specificity of the monoclonal antibody of the invention also for a urokinase-like plasminogen activator precursor (single chain precursor) produced by human epidermoid carcinoma cells ($A_{431}$ cells; Fabricant, De Larco, Todaro, 1977, Proc. Natl: Acad. Sci. USA, *74*, 565) was demonstrated and assay of the produced precursor performed by using the method of the invention.

The following examples further illustrate the present invention and must not be construed as limiting its scope.

Example 1
Preparation of anti-urokinase monoclonal antibody 5B4
a) Production of anti-urokinase monoclonal antibodies

Highly purified 54,000 Dalton urokinase 120,000 IU/mg (PERSOLV® RICHTER) is used to immunize 7-week old female Balb/C mice. 10 µg of this urokinase in complete Freund's adjuvant is injected into the peritoneum of the animals 60 days before fusion.

Another 10 µg of urokinase is given to mice 30 days later. Ten days before fusion, anti-urokinase antibody titer is measured by conventional ELISA method in blood samples taken from the tail veins and 5 days before fusion, a final booster of 10 µg of urokinase is given intravenously only to those animals having an antibody titer higher than 1:10,000. Spleens are then removed on the day of the fusion and spleen cells (about $1 \times 10^8$) are fused to $5 \times 10^7$ mouse myeloma cells X63-Ag8653 in 50% PEG 6000.

After fusion, which has been performed substantially following the method of C. Milstein and G. Köhler, the cells are resuspended in Dulbecco's Modified Eagle Medium (DMEM) supplemented with 10% fetal calf serum and HAT medium (0.1 mM Hypoxantine, 0.25 mM Aminopterine, 0.017 mM Thymidine, Flow Laboratories) and put onto 5 different Costar plates containing mouse macrophages (feeder layer).

The HAT medium is changed every 3 days and from day 10 after fusion the supernatants are tested in an ELISA immunoassay every 2—3 days for the presence of anti-urokinase monoclonal antibodies.

b) Screening of anti-urokinase monoclonal antibody-producing hybridomas by ELISA

According to a modification of the Perlman method (Immunochemistry, *8*, 873 (1971)), flexible 96 well microtitre plates (Dynatech) are coated with 50 µl/well of a 20 µg/ml solution of urokinase in phosphate buffered saline pH 7.2, (0.05 M sodium phosphate pH 7 which contains 0.5 M sodium chloride) and incubated for 1 h at room temperature. After a thorough washing with 0.05% Tween 20 phosphate buffer saline, the wells are saturated with 3% bovine serum albumin phosphate buffered saline, for 3 h at room temperature to avoid unspecific bindings. After washing carefully, 50 µl of supernatant hybridoma cultures (or the same volume of ascites fluids) are added to the wells and left to react two hours at room temperature. The plates are then washed and incubated with a 1:1000 dilution of rabbit-anti-mouse Ig conjugated with horseradish peroxidase (PI61, DAKO), for 90 min at room temperature. After thorough washing, the plates are incubated with 200 µl/well of 1 mg/ml solution of the chromogen o-phenylendiamine (SIGMA) in 0.1 M citrate buffer pH 5, 1.7 mM $H_2O_2$. The color is developed in 20 minutes and the optical density read at 492 nm against blanks with neither antigen nor antibody. Wells with color levels 4 times higher than the blanks are considered positive.

c) Mass cultivation of anti-urokinase producing hybridomas

Anti-urokinase antibody producing hybridomas are selected, cloned by limiting dilution, grown in mass cultures and injected into the peritoneum of Balb/c mice previously treated with 0.5 ml of pristane® (2,6,10,14-tetramethylpentadecane; Aldrich). Ascites fluids are collected 15 days later. The concentration of anti-urokinase monoclonal antibodies is in the range of 10—20 mg/ml. They are purified either by protein-A Sepharose or urokinase-Sepharose affinity chromatography.

d) Purification of anti-urokinase monoclonal antibodies by affinity chromatography on Sepharose®-urokinase

A sepharose-urokinase conjugate is prepared by reacting CNBr-activated Sepharose with highly purified urokinase (120,000 IU/mg). The coupling efficiency is of about 20 mg urokinase/ml swollen gel. Ascite fluids obtained as above (1 ml per producing hybridoma) are then applied to the Sepharose-urokinase affinity column (1.6 cm × 15 cm) pre-equilibrated in 0.01 M sodium phosphate buffer pH 8.0.

After rinsing with the same buffer, the selectively adsorbed anti-urokinase monoclonal antibodies are eluated with 0.1 M acetic acid pH 3.0. The antibody containing fractions are pooled and concentrated by ultrafiltration on PSDE 09005 Millipore membranes and stored frozen until use.

e) Evaluation of the purity and homogeneity of the monoclonal antibody preparations by gel-electrophoresis

The monoclonal antibody obtained above is submitted to SDS-PAGE (sodium dodecylphosphate polyacrylamide gel electrophoresis) according to the method described by W. K. Laemmli in Nature *227*, 680 (1970). Only the bands corresponding to the heavy and the light chains of the IgG are evident indicating that the above eluate contains pure immunoglobulins.

f) Determination of the affinity constant

Aliquots of a 20% Sepharose-urokinase suspension in phosphate buffered saline pH 7 (0.5 ml each) are added to increasing concentrations of purified anti-urokinase monoclonal antibody preparations (these preparations are those obtained according to point *d*, above, dialyzed in phosphate buffered saline pH 7). The samples are rotated end over end for two hours at room temperature. The controls received no resins.

The concentration of the unbound antibody is spectrophotometrically determined at 280 nm ($E_{1cm}^{1\%} = 14$), while the concentration of the bound antibody is calculated by difference from the total amount of added antibody. All binding data are elaborated according to Scatchard (Ann. N.Y. Acad. Sci., *51*, 660—672, 1949). The monoclonal antibody denominated 5B4 has an affinity constant of $1.42 \times 10^7$ l mole$^{-1}$.

Example 2

Immobilization of anti-urokinase monoclonal antibody 5B4 on activated agarose

Activated agarose (N-hydroxysuccinimide active ester derivative of cross-linked agarose beads having a charge free 10 atom long spacer arm; Affi-gel 10®, Bio-rad Inc.; 14 g, wet) is washed with 3 volumes of isopropyl alcohol and 3 volumes of cold distilled water in 20 minutes. The gel is then resuspended in 0.1 M sodium bicarbonate buffer pH 8 (final volume = 20 ml). Highly purified monoclonal antibody 5B4, as obtained above, in 0.1 M sodium bicarbonate buffer pH 8 (9.7 ml) is added to the gel suspension. After four hours at room temperature 1M aqueous ethanolamine hydrochloride at pH 8 (1.5 ml) is added to block the unreacted ester groups of the resin. The blocking reaction is completed in one hour at room temperature. The gel is then packed into a column, washed with 10 volumes of the coupling buffer (0.1 M aqueous sodium bicarbonate pH 8), then with 0.1 M aqueous acetic acid followed by 0.1 M aqueous acetic acid containing 1 M aqueous sodium chloride and lastly with 0.05 M aqueous sodium phosphate buffer pH 7 containing 0.5 M aqueous sodium chloride. The efficiency of the coupling is evaluated spectrophotometrically at 280 nm in the supernatant samples before and after reaction. These samples are brought to pH 2 before assaying to eliminate the interference of N-hydroxy succinimide in the readings.

2.5—4 mg of monoclonal antibody 5B4 were immobilized per ml of resin.

Example 3

a) Evaluation of the capacity of the 5B4-Agarose immunoadsorbent

The maximum capacity of 5B4-Agarose is evaluated on "pure" and crude preparations of Urokinase. A column of 5B4 Agarose (1.6 × 10 cm) is equilibrated in 0.5 M NaCl, 0.05 M Na=phosphate buffer pH 7 and loaded with "pure" urokinase (120,000 IU/mg) until esterolytic activity is detected in the effluent. The column is then washed with the equilibrating buffer and the enzyme is desorbed by eluting with 1 M NaCl, 0.1 M acetic acid. The total activity recovered in the bound fraction is taken as the maximum capacity of the column.

In the case of Urokinase (120,000 IU/mg) the capacity of 5B4 agarose was 216,000 IU/ml.

The same experiment was repeated using a crude preparation of urokinase (700 IU/mg) and a capacity of 174,000 IU/ml was obtained.

b) Immunoadsorption of HMW, LMW and 18,000 urokinase proteolytic fragment on 5B4 agarose

Purified HMW urokinase (30 mg) is incubated for 8 h in 1 ml of 50 mM sodium phosphate buffer pH 8 containing 0.2 M NaCl· HMW, LMW and 18,000 proteolytic fragments are obtained in a ratio of about 4:4:2, respectively. These products are separated and isolated by gel-filtration on a cross-linked controlled pore polydextran (Sephadex® G 100, superfine; 1.5 × 100 cm) equilibrated in 5 mM sodium phosphate buffer pH 4 containing 0.2 M NaCl and eluted with the same mixture at a flow rate of 3 ml/h. The eluted fractions containing the single products are then collected. Suspensions of scalar amounts of 5B4-agarose (prepared as described above) in phosphate buffered saline pH 7.5 containing 0.1% (v/v) Tween® 20 are added to test tubes containing 0.5 ml of HMW, LMW or 18,000 proteolytic fragment urokinase solutions (250 βg/ml each) in the same buffer and the final volume is brought to 1.5 ml with the same buffer.

All samples are rotated end over end for 1 h at room temperature and then centrifuged for 5 min at 2000 rpm. The unbound material is determined by reading the optical density of the supernatants at 280 nm; the bound material is determined by difference with the total added.

In this experiments, more than 90% of HMW urokinase and 18,000 proteolytic fragment are immunoadsorbed on 5B4-agarose whereas LMW urokinase is not immunoadsorbed.

Example 4

Purification of urokinase by immunoaffinity on 5B4-Agarose

A chromatography column (1.6 × 9 cm) is filled with the 5B4-Agarose immunoadsorbent as obtained according to example 2 and equilibrated with 0.5 M aqueous sodium chloride and 0.05 M aqueous phosphate buffer pH 7. A crude urinary concentrate is then loaded at a flow rate of 60 ml/h. After washing with the equilibrating buffer, urokinase is eluted with 1 M aqueous sodium chloride and 0.1 M aqueous acetic acid.

The urokinase containing fractions are pooled, neutralized and freeze-dried.

The specific activity of the recovered urokinase was always greater than 130,000 IU/mg.

The quantitative data are reported in the following table:

TABLE II

| FRACTION | VOL. | TOTAL FIBRINOLYTIC ACTIVITY [a] | % | TOTAL ESTEROLYTIC ACTIVITY [b] | TOTAL PROTEIN | SPEC. ACTIVITY | PURIF. FACTOR | IU/EU |
|---|---|---|---|---|---|---|---|---|
| | ml | IU | | EU | mg | IU/mg | | |
| CRUDE EXTRACT | 186 | 1772394 | 100 | 897 | 2007 | 883 | – | – |
| UNBOUND FRACTION (A) | 270 | 141791 | 8 | 164 | – | – | – | 846 |
| BOUND FRACTION (B) | 56 | 1374240 | 77.5 | 683 | 9.52 | 144353 | 163 | 2012 |

a) evaluated essentially following the method of the 1979 Addendum of the British Pharmacopoea

b) evaluated essentially following the method described Sherry et al., J. Lab. Clin. Med., 64, 145 (1964)

TABLE III

| | |
|---|---|
| Thromboplastinic contaminants: | zero-coagulation at 193 IU/ml plasma (zero-coagulation value of the National Heart and Lung Institute, U.S.A.: 80 IU/ml plasma) |
| Acute toxicity in mouse: | non toxic at 100,000 IU/kg, i.v. |
| Systemic tolerability in dog: | at 20,000 IU/kg there was no significant change in arterial blood pressure |

The HPLC analysis revealed the presence of high molecular weight (54,000) urokinase and the absence of low molecular weight (33,000) urokinase.

The purity of the obtained urokinase was also confirmed by gel electrophoresis on sodium dodecylsulfate polyacrylamide. Also this test, in fact, confirms that the urokinase product purified on 5B4-agarose is essentially 54,000 MW urokinase.

The experiments were repeated several times (more than 50 cycles) without any significant change in the results.

Consistently, 70—80% of the fibrinolytic activity loaded onto the column is detected in the bound fractions. When the unbound fractions are reloaded onto the column all the activity is detected in the flow-through suggesting that this activity may not be due to urokinase but rather to a specific proteases with fibrinolytic activity unrelated to urokinase.

By essentially following the above procedure but using urine (4:1) instead of a urine concentrate a urokinase having substantially the above characteristics is obtained.

Example 5

Preparation of $A_{431}$ cell supernatant

$A_{431}$ cells are grown to confluency in Dulbecco's modified Eagle medium containing 10% foetal calf serum (Flow Laboratories), 100 units/μl of penicillin, 100 μg/ml of streptomycin (Flow Labs.) and 2 mM of glutamine (Flow Labs.) in plastic culture flasks 150 cm$^2$ growth surface (NUNC). Then the supernatants are collected, centrifuged to eliminate the debris and stored at −80°C in the presence of a protease inhibitor (aprotinin, 10 μg/ml) until use.

Example 6

Purification of single-chain urokinase precursor from $A_{431}$ cells by means of immunoaffinity on 5B4-agarose

1.5 l of the centrifuged supernatant obtained according to the foregoing Example 5, is applied to a 5B4-agarose immunoadsorbent column (10 mm × 30 mm) obtained essentially following the procedure of Example 2, previously equilibrated with 0.15 M NaCl, 0.05 M Na-phosphate buffer, pH 7, at a flow rate of 40 mL/h. The column is washed with 50 ml of equilibration buffer and eluted with 1 M NaCl, 0.1 M acetic acid. Fractions are tested for the presence of urokinase antigen by double antibody sandwich immunoassay involving 5B4 monoclonal antibody (see Example 7 below). The obtained product (single chain urokinase precursor; (SC—UK)) is stored at −80°C at pH 4.8.

The fibrinolytic acctivity of this product is determined by the fibrin plate method (see Plough J. et al. (1957) Biochim Biophys Acta 24, 278) while the amidolytic activity on acetyl-lysyl-methyl-ester (ALME) is evaluated by a modification of the method of Sherry et al. (1964) J. Lab Clin Med 64, 145. Activation is obtained by mixing purified SC—UK (10 μg) with plasmin (4 μg) (Sigma) to a final volume of 0.4 ml in 0.5 M NaCl, 0.05 M Na-phosphate, pH 6, followed by incubation at room temperature. Samples are evaluated at different times for the amidolytic activity. The plasmin reaction is stopped by adding 100 μg of aprotinin (Richter) in 10 μl of buffer.

SDS-polyacrylamide-gel-electrophoresis (SDS-PAGE) in reducing and non reducing conditions was carried out according to Laemmli U.K. (1970) Nature 227, 680; immunoblotting experiments were carried out essentially as described previously (Salerno G. et al. (1984) Proc Natl Acad Sci USA 81, 110) using pure MAB 5B4, anti-urokinase and anti-tissue PA antisera in the immune reaction.

The immunoblotting pattern of MAB 5B4 indicates interactions with HMW-urokinase and with the proteolytic fragment (18,000) derived from the A-chain of HMW-urokinase. This indicates that the epitope must be located on the A-chain. The fact that no interaction is observed by reduction of HMW-urokinase with β-mercaptoethanol indicates that the conformational changes induced by the reductive reagent destroys the epitope.

The SDS-PAGE analysis of the product revealed a main band corresponding to a single polypeptide chain of 52,000. This value is slightly lower than the apparent molecular weight of human urokinase (54,000), as evaluated by SDS-PAGE. Immunoblotting of SC-UK with anti-urokinase monoclonal or polyclonal antibodies indicated the protein to be immunlogically related to urokinase. No reaction was observed with anti-tissue PA.

SC-UK was further characterized for its fibrinolytic and amidolytic actiity. The fibrinolytic activity of SC-UK, evaluated by the fibrin plate method, resulted to be 18,823 I.U./ml ($OD_{280}$), that is 7 times lower than the specific activity of pure 54,000 urokinase. Also the amidolytic activity on the synthetic substrate ALME resulted about 12 times lower than that of urokinase. However the amidolytic activity increased by eight fold after treatment with plasmin. Such an increase is associated to a conversion from the single-chain into a two-chain protein electrophoretically similar to human urokinase, confirming that SC-UK is a precursor of human urokinase.

Some quantitative data relating to this purification are reported in the following table.

TABLE IV

Purification of SC-UK from $A_{431}$ cell supernatants by affinity chromatography on a 5B4-agarose column

| Fraction | Volume (ml) | Urokinase (μg) | antigen[a] % | Fibrinolytic activity (U.I./ml) | Optical density 280 nm | I.U. $OD_{280}$ |
|---|---|---|---|---|---|---|
| Crude Material | 1100 | 265.0 | 100.0 | — | — | — |
| Unbound Fract. | 1150 | 6.7 | 2.5 | — | — | — |
| Bound Fract. | 6 | 176.7 | 66.7 | 640 | 0.034 | 18823 |

a) as measured by double antibody sandwich immunoassay

Example 7

Double-antibody sandwich ELISA

Flexible 96 well microtiter plates (FALCON 3912) are coated with 100 μl/well of purified anti-UK IgGs (a method for its preparation is reported below in Example 8) diluted 1:200 in PBS pH 7.2 (10 μg/ml of IgGs) and incubated for 1 h at 37°C. After washing with PBS-Tween 0.05%, the wells are contacted with 250 μl/well of PBS-BSA 3% for 2 h at room temperature for blocking uncoated plastic surface. After having discarded this solution, 100 μl of the appropriate dilution of standard urokinase in Dulbecco's modified Eagle medium (DMEM, Flow Labs.) supplemented with 10% FCS (foetal calf serum) or 100 μl of $A_{431}$ cell supernatant are added to each well and left to react overnight at 4°C. Microtiter plates were then washed with PBS-Tween 0.05% and incubated with 100 μl/well of 5B4-HRP conjugate (a method for its preparation is reported below in Example 9) diluted 1:100 in PBS-BSA 0.3%, Tween 0.05% for 30 minutes at 37°C.

After washing, the microtiter plates are incubated with 200 μl/well of a 1 mg/ml solution of o-phenylendiamine in 0.1 M sodium citrate pH 5 containing 5 mM $H_2O_2$ for 30 min at 37°C. The reaction is stopped with 4.5 M $H_2SO_4$ and the optical density read at 492 nm. The mean values of the optical density of the blank, the standard solutions and the test samples are separately evaluated. After subtracting the optical density value of the blank from the standard as well as the test samples, the urokinase content of the test samples is obtained by interpolating the corresponding competition ratio values on the calibration curve. The calibration curve is conveniently drawn on a semi-log paper plotting the optical density at 492 nm of each standard sample against the corresponding urokinase concentration. Validation experiments indicate that within — assay precision (CV) varies from 4% to 8% while between — assays precision (CV) is generally from 7 to 23%. Analytical recovery ranged from 87 to 114%. The application range is from 15 to 100 mg/ml of antigen.

Example 8

Purification of anti-urokinase immunoglobulin (IgGs) from an anti-UK conventional antiserum

Rabbit anti-UK antiserum was obtained according to usual immunization procedures by using highly purified 54,000 urokinase (about 120,000 IU/mg).

More particularly, healthy New Zealand white rabbits are immunized with 500 μg of highly purified 54,000 urokinase (about 120,000 IU/mg) in complete Freund's adjuvant.

Booster injections of the same amount of antigen in incomplete Freund's adjuvant are administered after 4, 6 and 8 weeks. Animals are bled weekly and anti-urokinase antibody titers are determined using anti-urokinase antiserum enzyme — Immunosassay. After 12 weeks animals are bled completely and IgG immunoglobulin fraction is purified by affinity chromatography on protein-A Sepharose.

One ml of the obtained antiserum is applied to a Protein A — Sepharose column (column size: 1.6 cm × 4 cm) previously equilibrated with 0.1 M sodium phosphate buffer pH 8.0 at a flow rate of 20 ml/h. After washing with the same buffer; IgGs are eluted with 0.1 M acetic acid pH 3. Fractions of 2 ml are collected and IgGs presence is monitored by enzyme immunoassay. Fractions containing IgGs are adjusted to pH 7.2 and stored frozen at −20°C until use.

Example 9

Labelling of monoclonal antibody 5B4 with horse-radish peroxidase

MAB 5B4 is labelled with horse-radish peroxidase (HRP), using glutaraldehyde as cross-linker reagent according to the following procedure. A 10 mg portion of HRP is dissolved in 200 µl of 0.1 M sodium phosphate pH 6.8 containing 1% glutaraldehyde and left to react at room temperature for 18 h. The reaction mixture is dialyzed against 0.9% NaCl and taken to 1 ml with the same solution. Then 1 ml of 0.9% NaCl containing 5 mg of MAB 5B4 and 200 µl of sodium carbonate 0.5 M pH 9.5 are added and allowed to react for 24 h at 4°C. To block the remaining active group, 100 µl of 0.2 M lysin were added and left to stand at room temperature for 2 h.

The mixture is then dialyzed overnight against 0.9% NaCl and gel filtered through a Sephacryl® S-200 column (column size: 1.6 cm × 100 cm) previously equilibrated with physiologic solution (flow rate 14 ml/ h). The chromatography is monitored by following the transmittance at 280 nm.

The peak eluted at the void volume of the column is collected and kept frozen at −20°C until use.

**Claims for the Contracting States: BE CH DE FR GB IT LI LU NL SE**

1. An anti-human urokinase monoclonal antibody having the following characteristics:
   a) it is a $IgG_1$
   b) it has an affinity constant of $(1.42 \pm 1.5) \times 10^7$ l mole$^{-1}$ for immobilized urokinase, when measured essentially following the procedure described by Tsapis et al. in Eur. J. Biochem. *64*, 369 (1976)
   c) it binds high molecular weight (54,000) urokinase and or a single chain precursor without binding the low molecular weight (33,000) urokinase
   d) it binds the 18,000 proteolytic fragment of the A-chain
   e) it does not cross-react with serum or urine enzymatically active endopeptidases other than urokinase.

2. An anti-human urokinase monoclonal antibody according to claim 1, further characterized in that
   a) it does not impair the enzymatic activity of urokinase
   b) it has an isoelectric point of about 5.75.

3. An anti-human urokinase monoclonal antibody as in claim 1 further characterized in that, when coupled to a suitable matrix, it is capable of binding the antigen at a pH between 4.5 and 8.0 even in the presence of 0.5 M aqueous sodium chloride and releasing the bound antibody at a pH between 3.0 and 4.0 in 0.5—1 M aqueous sodium chloride.

4. An immunoadsorbent for the purification of human urokinase and/or the single-chain urokinase precursor thereof which is obtained by linking the monoclonal antibody of claim 1 to a matrix selected from agarose, modified and activated agarose, cross-linked dextran, cellulose and carboxymethylcellulose.

5. An immunoadsorbent for the purification of human urokinase and/or the single-chain urokinase precursor thereof which is obtained by linking the monoclonal antibody of claim 1 to a matrix which is agarose.

6. An immunoadsorbent as in claim 4 or 5 further characterized in that when used in a purification process for urokinase it gives a urokinase product which has the following characteristics:
   a) it has a fibrinolytic titer higher than 130,000 IU/ml
   b) it is rich in high molecular weight urokinase and/or the single-chain urokinase precursor thereof
   c) it is practically devoid of low molecular weight urokinase
   d) it has a ratio fibrinolytic/esterasic activity higher than 2,000
   e) it is practically devoid of thromboplastinic contaminants (zero-coagulant activity at concentration of 200 IU/ml).

7. An immunoadsorbent according to claim 4 or 5 further characterized in that, when used for the purification of a single chain human urokinase precursor, it gives a human urokinase precursor having the following characteristics:
   a) it is a single-chain protein of MW 50,000—54,000
   b) it is almost devoid of amidolytic activity
   c) it has high fibrin activity
   d) it is not inactivated by plasma inhibitors
   e) it is converted into a two-chain active plasminogen activator by treatment with plasmin

8. A process for purifying 54,000 MW urokinase or the single-chain urokinase precursor thereof from biological sources which comprises contacting a biological source or a concentrate thereof with an immunoadsorbent of claim 4 or 5, at a pH between 4.5 and 8.0 in order to selectively bind the 54,000 MW urokinase or the single-chain urokinase precursor thereof to the immunoadsorbent, rinsing it with a buffered solution at a pH between 6 and 8 and releasing the bound antigen from the immunoadsorbent by eluting with an eluant solution, optionally containing 0.5—1 M aqueous sodium chloride at a pH between 3.0 and 4.0.

9. A process as in claim 8 wherein the binding of the antigen to the immunoadsorbent may be obtained also in the presence of aqueous sodium chloride in concentrations up to 0.5 M.

10. A process as in claim 9 further characterized in that a urokinase product is obtained which has the following characteristics:

a) it has a fibrinolytic titer higher than 130,000 IU/ml

b) it is rich in high molecular weight urokinase and/or the single-chain urokinase precursor thereof

c) it is practically devoid of low molecular weight urokinase

d) it has a ratio fibrinolytic/esterasic activity higher than 2,000

e) it is practically devoid of thromboplastinic contaminants (zero-coagulant activity at concentration of about 200 IU/ml).

11. Use of a monoclonal antibody of claim 1 or 2 for the purification of human urokinase or the single-chain urokinase precursor thereof by affinity chromatography.

12. Use of an immunoadsorbent of claim 4 or 5, for the purification of human urokinase or the single-chain urokinase precursor thereof by affinity chromatography.

13. A process for preparing anti-urokinase monoclonal antibody-producing hybridomas capable, upon culturing, of producing an anti-urokinase antibody as claimed in claim 1, which comprises carrying out somatic fusion of cells of a host immunized with 54,000 MW urokinase with myeloma cells of the same animal species in the presence of a fusion promoter, and screening for the desired producing hybridomas by means of enzyme-linked immunoassay with conventional anti-urokinase antiserum and class specificity, affinity constant and specific epitope determinations of the produced antibody.

14. A process as claimed in claim 13 wherein the somatic cells are mouse spleen cells and the myeloma cells are mouse myeloma cells.

15. A process as claimed in claim 13 wherein the myeloma cells are mouse myeloma cells known as X63-A$_g$ 8653.

16. A process as claimed in claim 13 wherein the host immunized with urokinase is a rabbit.

17. A process as claimed in claim 13 wherein the fusion promoter is a polyethylene glycol.

18. A process as claimed in claim 13 wherein the fusion promoter is polyethylene glycol 6000 (PEG 6000).

19. A process for preparing an anti-urokinase monoclonal antibody of claim 1 which comprises cultivating in a suitable host or culturing medium a hybridoma obtained according to claim 13.

20. Double antibody sandwich method for detecting or assaying an antigen selected from human urokinase and a precursor thereof which comprises:

a) causing a test solution containing the antigen to react with a first anti-urokinase antibody immobilized on a solid phase carrier to bind the antigen to the solid phase,

b) contacting with the bound antigen a solution containing a second anti-urokinase antibody having linked thereto a determinable marker, wherein one of the said first and second antibodies is a monoclonal antibody of claims 1 or 2

c) detecting or assaying the antigen in accordance with the extent to which the determinable marker has been bound to the solid phase carrier.

21. A method according to claim 20 wherein the "first" antibody is selected from an anti-urokinase antiserum, an anti-urokinase purified immunoglobulin G (IgGs) and a monoclonal antibody which is known to bind to a site of the antigen which is different from the binding site of the "second" monoclonal antibody and the second antibody is a monoclonal antibody according to claim 1 or 2.

22. A method according to claim 20 wherein the test solution is selected from diluted, undiluted or concentrated biological fluids and fermentation broths or extracts of genetically engineered microorganisms or cell cultures.

23. A method according to claim 20 wherein the determinable marker linked to the "second" antibody is an enzyme.

24. A method according to claim 20 wherein the determinable marker linked to the "second" antibody is horseradish peroxidase.

25. A method according to claim 20 wherein the solid phase carrier is selected from particles of cellulose, polyacrylamide, cross-linked dextran, silicone rubber, microcrystalline glass and plastics.

26. A method according to claim 20 wherein the solid phase carrier is a preformed plastic material such as a polystyrene, polypropylene or polyvinyl tube, disc or microplate.

27. A method according to claim 20 wherein the solid phase carrier is a preformed polyvinyl microplate.

28. A biological test kit for assaying (HMW) human urokinase or a single chain precursor thereof which includes:

a) a solid-phase carrier having immobilized on its surface a first anti-urokinase antibody, and

b) a solution or a lyophilized preparation of a second anti-urokinase antibody having a determinable marker bound thereto, wherein one of said first and second antibody is a monoclonal antibody of claim 1.

29. A kit as claimed in claim 28 wherein said second antibody is a monoclonal antibody of claim 1.

30. A kit as claimed in claim 28 wherein said second antibody is a monoclonal antibody of claim 1 and said first antibody is selected from an anti-urokinase antiserum, anti-urokinase purified IgG(s) and a monoclonal antibody which binds an antigen site different from the binding site of said second antibody.

31. A kit as claimed in claim 28 wherein one of said first and second antibody is monoclonal antibody of claim 1 and the other is a monoclonal antibody known to bind to a different site of the antigen.

14

32. A kit as claimed in claim 28 wherein said determinable marker is an enzyme.

33. A kit as claimed in claim 28 wherein said determinable marker is horseradish peroxidase.

**Claims for the Contracting State: AT**

1. A process for preparing anti-urokinase monoclonal antibody-producing hybridomas capable, upon culturing, of producing an anti-urokinase antibody having the following characteristics:

a) it is an $IgG_1$

b) it has an affinity constant of $(1.42 \pm 1.5) \times 10^7$ l mole$^{-1}$ for immobilized urokinase, when measured essentially following the procedure described by Tsapis et al. in Eur. J. Biochem. *64*, 369 (1976)

c) it binds high molecular weight (54,000) urokinase and/or the single-chain urokinase precursor without binding the low molecular weight (33,000) urokinase

d) it binds the 18,000 proteolytic fragment of the A-chain of urokinase

e) it does not cross-react with serum or urine enzymatically active endopeptidases other than urokinase, which comprises carrying out somatic fusion of cells of a host immunised with 54,000 MW urokinase with myeloma cells of the same animal species in the presence of a fusion promoter, and screening for the desired producing hybridomas by means of enzyme-linked immunoassay with conventional anti-urokinase antiserum and class specificity, affinity constant and specific epitope characteristics of the produced antibody.

2. A process as claimed in claim 1 wherein the somatic cells are mouse spleen cells and the myeloma cells are mouse myeloma cells.

3. A process as claimed in claim 1 wherein the myeloma cells are mouse myeloma cells known as X63-$A_g$ 8653.

4. A process as claimed in claim 1 wherein the host immunized with urokinase is a rabbit.

5. A process as claimed in claim 1 wherein the fusion promoter is a polyethylene glycol.

6. A process as claimed in claim 5 wherein the fusion promoter is polyethylene glycol 6000 (PEG 6000).

7. A process for preparing an anti-urokinase monoclonal antibody of claim 1 which comprises cultivating in a suitable host or culturing medium an hybridoma obtained according to claim 1.

8. A process for preparing an anti-human urokinase monoclonal antibody according to claim 1, further characterized in that

a) it does not impair the enzymatic activity of urokinase

b) it has an isoelectric point of about 5.75.

9. A process as claimed in claim 1 for preparing anti-human urokinase monoclonal antibody further characterized in that, when coupled to a suitable matrix, it is capable of binding the antigen at a pH between 4.5 and 8.0 even in the presence of 0.5 M aqueous sodium chloride and releasing the bound antibody at a pH between 3.0 and 4.0 in 0.5—1 M aqueous sodium chloride.

10. A process for purifying 54,000 MW urokinase or the single-chain urokinase precursor thereof from biological sources which comprises contacting a biological source or concentrate thereof with an immunoadsorbent obtained by linking an anti-urokinase antibody having the characteristics of the monoclonal antibody of claim 1 to a maxtrix selected from agarose, modified and activated agarose, cross-linked dextran, cellulose and carboxymethylcellulose, at a pH between 4.5 and 8.0 in order to selectively bind the 54,000 MW urokinase or the single-chain urokinase precursor thereof to the immunoadsorbent, rinsing it with a buffered solution at a pH between 6 and 8 and releasing the bound antigen from the immunoadsorbent by eluting with an eluant solution, optionally containing 0.5—1 M aqueous sodium chloride at a pH between 3.0 and 4.0.

11. A process as in claim 10 wherein the binding of the antigen to the immunoadsorbent may be obtained also in the presence of aqueous sodium chloride in concentrations up to 0.5 M.

12. A process as in claim 10 further characterized in that a urokinase product is obtained which has the following characteristics:

a) it has a fibrinolytic titer higher than 130,000 IU/ml

b) it is rich in high molecular weight urokinase or the single-chain urokinase precursor thereof

c) it is practically devoid of low molecular weight urokinase

d) it has a ratio fibrinolytic/esterasic activity higher than 2,000

e) it is practically devoid of thromboplastinic contaminants (zero-coagulant activity at concentration of about 200 IU/ml).

13. A process as in claim 10 further characterized in that a human urokinase precursor is obtained having the following characteristics:

a) it is a single-chain protein of MW 50,000—54,000

b) it is almost devoid of amidolytic activity

c) it has high fibrin activity

d) it is not inactivated by plasma inhibitors

e) it is converted into a two-chain active plasminogen activator by treatment with plasmin.

14. A process as in claim 10 wherein the immunoadsorbent for the purification of human urokinase and/or the single-chain urokinase precursor thereof is obtained by linking the monoclonal antibody described in claim 1 to a matrix which is agarose.

15. A process as in claim 10 wherein the monoclonal antibody is further chracterized in that

a) it does not impair the enzymatic activity of urokinase

b) it has an isoelectric point of about 5.75.

16. Double antibody sandwich method for detecting or assaying an antigen selected from human urokinase and a precursor thereof which comprises:

a) causing a test solution containing the antigen to react with a first anti-urokinase antibody immobilized on a solid phase carrier to bind the antigen to the solid phase,

b) contacting with the bound antigen a solution containing a second anti-urokinase antibody haing linked thereto a determinable marker, wherein one of the said first and second antibodies is a monoclonal antibody having the characteristics of the monoclonal antibody of claim 1

c) detecting or assaying the antigen in accordance with the extent to which the determinable marker has been bound to the solid phase carrier.

17. A method as in claim 1 wherein said monoclonal antibody is further characterized in that:

— it does not impair the enzymatic activity of urokinase

— it has an isoelectric point of about 5.75.

18. A method according to claim 1 wherein the "first" antibody is selected from an anti-urokinase antiserum, an anti-urokinase purified immunoglobulin G and a monoclonal antibody which is known to bind to a site of the antigen which is different from the binding site of the "second" monoclonal antibody and the second antibody is a monoclonal antibody according to claim 1 or 2.

19. A method according to claim 1 wherein the test solution is selected from diluted, undiluted or concentrated biological fluids and fermentation broths or extracts of genetically engineered microorganisms or cell cultures.

20. A method according to claim 1 wherein the determinable marker linked to the "second" antibody is an enzyme.

21. A method according to claim 1 wherein the determinable marker linked to the "second" antibody is horseradish peroxidase.

22. A method according to claim 1 wherein the solid phase carrier is selected from particles of cellulose, polyacrylamide, cross-linked dextran, silicone rubber, microcrystalline glass and plastics.

23. A method according to claim 1 wherein the solid phase carrier is a preformed plastic material such as a polystyrene, polypropylene or polyvinyl tube, disc or microplate.

24. A method according to claim 1 wherein the solid phase carrier is a preformed polyvinyl microplate.

**Patentansprüche für die Vertragsstaaten: BE CH DE FR GB IT LI LU NL SE**

1. Monoklonarer Antihumanurokinase-Antikörper, gekennzeichnet durch die folgenden Eigenschaften:

a) er ist ein IgG$_1$

b) er besitzt eine Affinitätkonstante von $(1,42 \pm 1,5) \times 10^7$ 1 mol$^{-1}$ gegenüber immobilisierter Humanurokinase bei Messung im wesentlichen nach dem Verfahren beschrieben von Tsapis et al. in Eur. J. Biochem. *64*, 369 (1976)

c) er bindet hochmolekulare (54.000) Urokinase und/oder den einkettigen Urokinasevorläufer ohne die niedermoleculare (33.000) Urokinase zu binden

d) er bindet das proteolytische Fragment von 18.000 der A-Kette der Urokinase

e) er zeigt keine Kreuzreaktion mit enzymatisch aktiven Serum- oder Urinendopeptidasen außer Urokinase.

2. Monoklonaler Antihumanurokinase-Antikörper nach Anspruch 1, dadurch gekennzeichnet, daß

a) er die enzymatische Aktivität der Urokinase nicht beeinträchtigt

b) er weiterhin einen isoelektrischen Punkt von etwa 5,75 besitzt.

3. Monoklonaler Antihumanurokinase-Antikörper nach Anspruch 1, dadurch gekennzeichnet, daß er weiterhin bei Kopplung an eine geeignete Matrix geeignet ist, das Antigen bei einem pH zwischen 4,5 und 8,0 sogar in Anwesenheit einer 0,5 m wäßrigen Natriumchloridlösung zu binden und den gebundenen Antikörper be einem pH zwischen 3,0 und 4,0 in 0,5—1 m wäßrigen Natriumchloridlösung freizusetzen.

4. Immunoadsorbens zur Reinigung von Humanurokinase und/oder ihres einkettigen Urokinasevorläufers, dadurch gekennzeichnet, daß es durch Koppeln des monoklonalen Antikörpers nach Anspruch 1 an eine Matrix ausgewählt aus Agarose, modifizierte und aktivierte Agarose, quervernetztes Dextran, Cellulose und Carboxymethylcellulose erhalten worden ist.

5. Immunoadsorbens zur Reinigung von Humanurokinase oder ihres einkettigen Vorläufers, dadurch gekennzeichnet, daß es durch Koppeln des monoklonalen Antikörpers nach Anspruch 1 an Agarose als Matrix erhalten worden ist.

6. Immunoadsorbens nach Anspruch 4 oder 5, dadurch gekennzeichnet, daß es weiterhin bei Verwendung in einem Reinigungsverfahren für Urokinase ein Urokinaseprodukt mit den folgenden Eigenschaften ergibt:

a) es besitzt einen fibrinolytischen Titer größer als 130.000 IU/ml

b) es ist reich an hochmolekularer Urokinase und/oder an ihrem einkettigen Urokinasevorläufer

c) es ist praktisch frei von niedermolekularer Urokinase

d) das Verhältnis fibrinolytischer/Esteraseaktivität ist größer als 2.000

e) est ist praktisch frei von thromboplastinischen Verunreinigungen (Null-Koagulansaktivität bei einer Konzentration von 200 IU/ml).

7. Immunoadsorbens nach Anspruch 4 oder 5, dadurch gekennzeichnet, daß bei Verwendung zur Reinigung eines einkettigen Urokinasevorläufers es einen Humanurokinasevorläufer mit den folgenden Eigenschaften ergibt:

a) er ist ein einkettiges Protein mit einem Molekulargewicht von 50.000 bis 54.000

b) er ist fast frei von amidolytischer Aktivität

c) er besitzt eine hohe Fibrinaktivität

d) er wird durch Plasmainhibitoren nicht inaktiviert

e) er wird durch Behandlung mit Plasmin in einen zweikettigen aktiven Plasminogenaktivator umgewandelt.

8. Verfahren zur Reinigung von Urokinase mit einem Molekulargewicht von 54.000 oder ihres einkettigen Urokinasevorläufers aus biologischen Quellen, dadurch gekennzeichnet, daß eine biologische Quelle oder ihr Konzentrat mit einem Immunoadsorbenten nach Anspruch 4 oder 5 bei einem pH zwischen 4,5 und 8,0 in Kontakt gebracht wird, um selektiv die Urokinase mit dem Molekulargewicht von 54.000 oder ihren einkettigen Urokinasevorläufer an den Immunoadsorbenten zu binden, er mit einer gepufferten Lösung bei einem pH zwischen 6 und 8 gespült wird und das gebundene Antigen aus dem Immunoadsorbenten durch Elution mit einer Elutionslösung, die gegebenenfalls 0,5 bis 1 m wasserhaltiges Natriumchlorid bei einem pH zwischen 3,0 und 4,0 enthält, freigesetzt wird.

9. Verfahren nach Anspruch 8, dadurch gekennzeichnet, daß das Antigen auch in Gegenwart von wasserhaltigem Natriumchlorid in Konzentrationen bis zu 0,5 m an den Immunoadsorbenten gebunden werden kann.

10. Verfahren nach Anspruch 9, dadurch gekennzeichnet, daß weiterhin das erhaltene Urokinaseprodukt die folgenden Eigenschaften besitzt:

a) es besitzt einen fibrinolytischen Titer größer als 130.000 IU/ml

b) es ist reich an hochmolekularer Urokinase oder ihres einkettigen Urokinasevorläufers

c) es ist praktischfrei von niedermolekularer Urokinase

d) das Verhältnis fibrinolytischer/Esteraseaktivität ist größer als 2.000

e) es ist praktisch frei von thromboplastinischen Verunreinigungen (Null-Koagulansaktivität bei einer Konzentration von 200 IU/ml).

11. Verwendung eines monoklonalen Antikörpers nach Anspruch 1 oder 2 zur Reinigung der Humanurokinase oder ihres einkettigen Urokinasevorläufers mittels Affinitätschromatographie.

12. Verwendung eines Immunoadsorbenten nach Anspruch 4 oder 5 zur Reinigung der Humanurokinase oder ihres einkettigen Urokinasevorläufers mittels Affinitätschromatographie.

13. Verfahren zur Herstellung von monoklonale Antiurokinase-Antikörper produzierenden Hybridomen, die nach Kultivierung einen Antiurokinaseantikörper nach Anspruch 1 produzieren können, dadurch gekennzeichnet, daß eine somatische Fusion von Zellen eines Wirts, der mit Urokinase mit einem Molekulargewicht von 54.000 immunisiert worden ist, mit Myelomzellen der gleichen Tierspezies in Gegenwart eines Fusionspromotors durchgeführt wird, daß auf die gewünschten produzierenden Hybridome mittels Enzymlink-Immunoassay mit üblichem Antiurokinaseantiserum und Gruppenspezifität gescreent wird und daß die Affinitätskonstante und das spezifische Epitop des produzierten Antikörpers bestimmt werden.

14. Verfahren nach Anspruch 13, dadurch gekennzeichnet, daß die somatischen Zellen Milzzellen aus Mäusen und die Myelomzellen Myelomzellen aus Mäusen sind.

15. Verfahren nach Anspruch 13, dadurch gekennzeichnet, daß die Myelomzellen Myelomzellen der Maus, bekannt als X63-Ag 8653 sind.

16. Verfahren nach Anspruch 13, dadurch gekennzeichnet, daß der mit Urokinase immunisierte Wirt ein Kaninchen ist.

17. Verfahren nach Anspruch 13, dadurch gekennzeichnet, daß der Fusionspromotor Polyethylenglykol ist.

18. Verfahren nach Anspruch 13, dadurch gekennzeichnet, daß der Fusionspromotor Polyethylenglykol 6000 (PEG 6000) ist.

19. Verfahren zur Herstellung eines monoklonalen Antiurokinase-Antikörpers nach Anspruch 1, dadurch gekennzeichnet, daß ein nach Anspruch 13 erhaltenes Hybridom in einem geeigneten Wirt oder Kulturmedium kultiviert wird.

20. Doppeltes Antikörper-Sandwichverfahren zum Nachweis oder zur Untersuchung auf ein Antigen, ausgewählt aus Humanurokinase und ihrem Vorläufer, dadurch gekennzeichnet, daß

a) man eine Testlösung, die das Antigen enthält, mit einem ersten Antiurokinaseantikörper, der auf einem Festphasenträger immobilisiert ist, reagieren läßt, um das Antigen an die Festphase zu binden,

b) man das gebundene Antigen mit einer Lösung in Kontakt bringt, die einen zweiten Antiurokinaseantikörper, an den ein bestimmbarer Marker geknüpft ist, enthält, wobei einer der ersten und zweiten Antikörper ein monoklonaler Antikörper nach Anspruch 1 oder 2 ist,

c) man das Antigen detektiert oder auf es untersucht gemäß dem Ausmaß, in dem der bestimmbare Marker an den Festphasenträger gebunden worden ist.

21. Verfahren nach Anspruch 20, dadurch gekennzeichnet, daß der "erste" Antikörper aus einem Antiurokinaseantiserum, einem gereinigten Antiurokinase-Immunglobulin G (IgGs) und einem monoklonalen Antikörper, von dem bekannt ist, daß er an eine Stelle des Antigens, die sich von der Bindungsstelle des "zweiten" monoklonalen Antikörpers unterscheidet, bindet, ausgewählt ist, und daß der zweite Antikörper ein monoklonaler Antikörper nach Anspruch 1 oder 2 ist.

22. Verfahren nach Anspruch 20, dadurch gekennzeichnet, daß die Testlösung ausgewählt ist aus verdünnten, unverdünnten oder konzentrierten biologischen Flüssigkeiten und Fermentationslösungen oder Extrakten aus genetisch manipulierten Mikroorganismen oder Zellkulturen.

23. Verfahren nach Anspruch 20, dadurch gekennzeichnet, daß der mit dem "zweiten" Antikörper verknüpfte bestimmbare Marker ein Enzym ist.

24. Verfahren nach Anspruch 20, dadurch gekennzeichnet, daß der mit dem "zweiten" Antikörper verknüpfte bestimmbare Marker Meerrettichpeoxidase ist.

25. Verfahren nach Anspruch 20, dadurch gekennzeichnet, daß der Festphasenträger aus Cellulose-, Polyacrylamid-, quervernetzten Dextran-, Silikongummi-, mikrokristallinen Glas- und Kunststoffteilchen ausgewählt ist.

26. Verfahren nach Anspruch 20, dadurch gekennzeichnet, daß der Festphasenträger ein vorgeformtes Kunststoffmaterial wie z.B. ein Polystyrol-, Polypropylen- oder Polyvinyl-Rohr, -Scheibchen oder -Mikroplatte ist.

27. Verfahren nach Anspruch 20, dadurch gekennzeichnet, daß der Festphasenträger eine vorgeformte Polyvinylmikroplatte ist.

28. Biologischer Testkit zur Bestimmung von Humanurokinase (HMW) oder ihres einkettigen Vorläufers, dadurch gekennzeichnet, daß er

a) einen Festphasenträger, auf dessen Oberfläche ein erster Antiurokinaseantikörper immobilisiert ist, und

b) eine Lösung oder ein Lyophilisat eines zweiten Antiurokinaseantikörpers, an den ein bestimmbarer Marker geknüpft ist, wobei einer der ersten und zweiten Antikörper ein monoklonaler Antikörper nach Anspruch 1 ist, umfaßt.

29. Kit nach Anspruch 28, dadurch gekennzeichnet, daß der zweite Antikörper ein monoklonaler Antikörper nach Anspruch 1 ist.

30. Kit nach Anspruch 28, dadurch gekennzeichnet, daß der zweite Antikörper ein monoklonaler Antikörper nach Anspruch 1 ist und daß der erste Antikörper ausgewählt wird aus einem Antiurokinaseantiserum, einem gereinigten Antiurokinase-IgG(s) und einem monoklonalen Antikörper, der an eine antigene Stelle, die sich von der Bindungsstelle des zweiten Antikörpers unterscheidet, bindet.

31. Kit nach Anspruch 28, dadurch gekennzeichnet, daß einer der ersten und zweiten Antikörper ein monoklonaler Antikörper nach Anspruch 1 ist und der andere ein monoklonaler Antikörper, von dem bekannt ist, daß er an eine andere Stelle des Antigens bindet, ist.

32. Kit nach Anspruch 28, dadurch gekennzeichnet, daß der bestimmbare Marker ein Enzym ist.

33. Kit nach Anspruch 28, dadurch gekennzeichnet, daß der bestimmbare Marker Meerrettichperoxidase ist.

## Patentansprüche für den Vertragsstaat: AT

1. Verfahren zur Herstellung von monoklonale Antiurokinase-Antikörper produzierenden Hybridomen, die nach Kultivierung einen Antiurokinaseantikörper mit den folgenden Eigenschaften

a) er ist ein $IgG_1$

b) er besitzt eine Affinitätkonstante von $(1,42 \pm 1,5) \times 10^7$ 1 $mol^{-1}$ gegenüber immobilisierter Humanurokinase bei Messung im wesentlichen nach dem Verfahren, beschrieben von Tsapis et al. in Eur. J. Biochem. 64, 369 (1976)

c) er bindet hochmolekulare Urokinase (54.000) und/oder den einkettigen Urokinasevorläufer ohne die niedermolekulare Urokinase (33.000) zu binden

d) er bindet das proteolytische Fragment von 18.000 der A-Kette der Urokinase

e) er zeigt keine Kreuzreaktion mit enzymatisch aktiven Serum- oder Urinendopeptidasen außer Urokinase produzieren können, dadurch gekennzeichnet, daß eine somatische Fusion von Zellen eines Wirts, der mit Urokinase mit einem Molekulargewicht von 54.000 immunisiert wurde, mit Myelomzellen der gleichen Tierspezies in Gegenwart eines Fusionspromotors durchgeführt wird, daß auf die gewünschten produzierenden Hybridome mittels eines Enzymlink-Immunoassays mit üblichen Antiurokinaseantiserum und Gruppenspezifität gescreent wird, und daß die Affinitätskonstante und das spezifische Epitop des produzierten Antikörpers charakterisiert werden.

2. Verfahren nach Anspruch 1, dadurch gekennzeichnet, daß die somatischen Zellen Milzzellen aus Mäusen und die Myelomzellen Myelomzellen aus Mäusen sind.

3. Verfahren nach Anspruch 1, dadurch gekennzeichnet, daß die Myelomzellen Myelomzellen der Maus, bekannt als $X63-A_g$ 8653 sind.

4. Verfahren nach Anspruch 1, dadurch gekennzeichnet, daß der mit Urokinase immunisierte Wirt ein Kaninchen ist.

5. Verfahren nach Anspruch 1, dadurch gekennzeichnet, daß der Fusionspromotor Polyethylenglykol ist.

EP 0 192 675 B1

6. Verfahren nach Anspruch 5, dadurch gekennzeichnet, daß der Fusionspromotor Polyethylenglykol 6000 (PEG 6000) ist.

7. Verfahren zur Herstellung eines monoklonalen Antiurokinase-Antikörpers nach Anspruch 1, dadurch gekennzeichnet, daß ein nach Anspruch 1 erhaltenes Hybridom in einem geeigneten Wirt oder Kulturmedium kultiviert wird.

8. Verfahren zur Herstellung eines monoklonalen Antihumanurokinase-Antikörpers nach Anspruch 1, dadurch gekennzeichnet, daß

a) er die enzymatische Aktivität der Urokinase nicht beeinträchtigt

b) er einen isoelektrischen Punkt von etwa 5,75 besitzt.

9. Verfahren nach Anspruch 1 zur Herstellung von monoklonalem Antihumanurokinase-Antikörper, dadurch gekennzeichnet, daß er bei Kopplung an eine geeignete Matrix das Antigen bei einem pH zwischen 4,5 und 8,0 sogar in Gegenwart einer 0,5 m wäßrigen Natriumchloridlösung binden und den gebundenen Antikörper bei einem pH zwischen 3,0 und 4,0 in 0,5—1 m wäßrigen Natriumchloridlösung freisetzen kann.

10. Verfahren zur Reinigung von Urokinase mit einem Molekulargewicht von 54.000 oder ihres einkettigen Urokinasevorläufers aus biologischen Quellen, dadurch gekennzeichnet, daß eine biologische Quelle oder ihr Konzentrat mit einem Immunoadsorbenten bei einem pH zwischen 4,5 und 8,0 in Kontakt gebracht wird, der durch Koppeln eines Antiurokinaseantikörpers mit den Eigenschaften des monoklonalen Antikörpers nach Anspruch 1 an eine Matrix, ausgewählt aus Agarose, modifizierter und aktivierter Agarose, quervernetztem Dextran, Cellulose und Carboxymethylcellulose erhalten worden ist, um selektiv die Urokinase mit dem Molekulargewicht von 54.000 oder ihren einkettigen Urokinasevorläufer an den Immunoadsorbenten zu binden, er mit einer gepufferten Lösung bei einem pH zwischen 6 und 8 gespült wird und das gebundene Antigen aus dem Immunoadsorbenten durch Elution mit einer Elutionslösung, die gegebenenfalls 0,5 bis 1 m wasserhaltiges Natriumchlorid bei einem pH zwischen 3,0 und 4,0 enthält, freigesetzt wird.

11. Verfahren nach Anspruch 10, dadurch gekennzeichnet, daß das Antigen auch in Gegenwart von wäßrigem Natriumchlorid in Konzentrationen bis zu 0,5 m an den Immunoadsorbenten bindet.

12. Verfahren nach Anspruch 10, dadurch gekennzeichnet, daß das erhaltene Urokinaseprodukt die folgenden Eigenschaften besitzt:

a) es besitzt einen fibrinolytischen Titer größer als 130.000 IU/ml

b) es ist reich an hochmolekularer Urokinase oder ihres einkettigen Urokinasevorläufers

c) es ist praktisch frei von niedermolekularer Urokinase

d) das Verhältnis fibrinolytischer/Esteraseaktivität ist größer als 2.000

e) est ist praktisch frei von thromboplastinischen Verunreinigungen (Null-Koagulansaktivit bei einer Konzentration von 200 IU/ml).

13. Verfahren nach Anspruch 10, dadurch gekennzeichnet, daß der erhaltene Humanurokinasevorläufer die folgenden Eigenschaften besitzt:

a) er ist ein einkettiges Protein mit einem Molekulargewicht von 50.000 bis 54.000

b) er ist fast frei von amidolytischer Aktivität

c) er besitzt eine hohe Fibrinaktivität

d) er wird durch Plasmainhibitoren nicht inaktiviert

e) er wird durch Behandlung mit Plasmin in einen zweikettigen aktiven Plasminogenaktivator umgewandelt.

14. Verfahren nach Anspruch 10, dadurch gekennzeichnet, daß der Immunoadsorbent zur Reinigung von Humanurokinase und/oder ihres einkettigen Urokinasevorläufers durch Koppeln des monoklonalen Antikörpers nach Anspruch 1 an Agarose als Matrix erhalten worden ist.

15. Verfahren nach Anspruch 10, dadurch gekennzeichnet, daß der monoklonale Antikörper

a) die enzymatische Aktivität der Urokinase nicht beeinträchtigt,

b) einen isoelektrischen Punkt von etwa 5,75 besitzt.

16. Doppeltes Antikörper-Sandwichverfahren zum Detektieren oder zur Untersuchung auf ein Antigen, ausgewählt aus Humanurokinase und ihrem Vorläufer, dadurch gekennzeichnet, daß

a) man eine Testlösung, die das Antigen enthält, mit einem ersten Antiurokinaseantikörper, der auf einem Festphasenträger immobilisiert ist, reagieren läßt, um das Antigen an die fest phase zu binden,

b) man das gebundene Antigen mit einer Lösung in Kontakt bringt, die einen zweiten Antiurokinaseantikörper, an die einbestimmbarer Marker geknüpft ist, enthält, worin einer der ersten und zweiten Antikörper ein monoklonaler Antikörper mit den Eigenschaften des monoklonalen Antikörpers nach Anspruch 1 ist,

c) man das Antigen detektiert oder auf es untersucht je nach dem Ausmaß, in dem der bestimmbare Marker an den Festphasenträger gebunden worden ist.

17. Verfahren nach Anspruch 1, dadurch gekennzeichnet, daß der monoklonale Antikörper die enzymatische Aktivität der Urokinase nicht beeinträchtigt und einen isoelektrischen Punkt von etwa 5,75 besitzt.

18. Verfahren nach Anspruch 1, dadurch gekennzeichnet, daß der "erste" Antikörper ausgewählt wird aus einem Antiurokinaseantiserum, einem gereinigten Antiurokinase-Immunglobulin G und einem monoklonalen Antikörper, von dem bekannt, daß er an eine Stelle des Antigens bindet, die sich von der Bindungsstelle des "zweiten" monklonalen Antikörpers unterscheidet, und daß der zweite Antikörper ein

19

monoklonaler Antikörper nach Anspruch 1 oder 2 ist.

19. Verfahren nach Anspruch 1, dadurch gekennzeichnet, daß die Testlösung ausgewählt ist aus verdünnten, unverdünnten oder konzentrierten biologischen Flüssigkeiten und Fermentationslösungen oder Extrakten aus genetische manipulierten Mikroorganismen oder Zellkulturen.

20. Verfahren nach Anspruch 1, dadurch gekennzeichnet, daß der bestimmbare Marker, der mit dem "zweiten" Antikörper verknüpft ist ein Enzym ist.

21. Verfahren nach Anspruch 1, dadurch gekennzeichnet, daß der bestimmbare Marker, der mit dem "zweiten" Antikörper verknüpft ist, Meerrettichperoxidase ist.

22. Verfahren nach Anspruch 1, dadurch gekennzeichnet, daß der Festphasenträger aus Cellulose-, Polyacrylamid-, quervernetzten Dextran-, Silikongummi-, mikrokristallinen Glas- und Kunststoffteilchen ausgewählt ist.

23. Verfahren nach Anspruch 1, dadurch gekennzeichnet, daß der Festphasenträger ein vorgeformtes Kunststoffmaterial wie z.B. ein Polystyrol-, Polypropylen- oder Polyvinyl-Rohr, -Scheibchen oder -Mikroplatte ist.

24. Verfahren nach Anspruch 1, dadurch gekennzeichnet, daß der Festphasenträger eine vorgeformte Polyvinylmikroplatte ist.

## Revendications pour les Etats contractants: BE CH DE FR GB IT LI LU NL SE

1. Anticorps monoclonal anti-urokinase humaine ayant les caractéristiques suivantes:
   a) il s'agit d'une IgG$_1$
   b) il a une constante d'affinité de $(1,42 \pm 1,5) \times 10^7$ 1 mole$^{-1}$ pour l'urokinase humaine immobilisée, la mesure étant effectuée essentiellement par le mode opératoire décrit par Tsapis et al. dans Eur. J. Biochem. *64*, 369 (1976)
   c) il fixe l'urokinase à masse moléculaire élevée (54.000) et/ou le précurseur d'urokinase à chaîne unique sans fixation de l'urokinase à faible masse moléculaire (33.000)
   d) il fixe le fragment protéolytique 18.000 de la chaîne A de l'urokinase
   e) il ne subit pas de réaction croisée avec les endopeptidases enzymtiquement actives sur le sérum ou l'urine autres que l'urokinase.

2. Anticorps monoclonal anti-urokinase humaine selon la revendication 1, caractérisé en outre en ce que
   a) il ne perturbe pas l'activité enzymatique de l'urokinase
   b) il a un point isoélectrique d'environ 5,75.

3. Anticorps monoclonal anti-urokinase humaine selon la revendication 1, caractérisé en outre en ce que, quand il est couplé à une matrice appropriée, il est capable de fixer l'antigène à un pH de 4,5 à 8,0 même en présence de chlorure de sodium aqueux 0,5 M, et de libérer l'anticorps fixé à un pH de 3,0 à 4,0 dans du chlorure de sodium aquex 0,5—1 M.

4. Immuno-adsorbant pour la purification de l'urokinase humaine et/ou de son précurseur d'urokinase à chaîne unique, que l'on obtient en liant l'anticorps monoclonal de la revendication 1 à une matrice choisie parmi l'agarose, l'agarose modifée et activée, le dextrane réticulé, la cellulose et la carboxyméthylcellulose.

5. Immuno-adsorbant pour la purification de l'urokinase humaine et/ou de son précurseur d'urokinase à chaîne unique, que l'on obtient en liant l'anticorps monoclonal de la revendication 1 à une matrice constituée d'agarose.

6. Immuno-adsorbant selon la revendication 4 ou 5, caractérisé en outre en ce que, quand on l'utilise dans un procédé de purification de l'urokinase, il donne un produit urokinase qui a les caractéristiques suivantes:
   a) il a un titre fibrinolytique supérieur à 130.000 UI/ml
   b) il est riche en urokinase à masse moléculaire élevée et/ou en son précurseur d'urokinase à chaîne unique
   c) il est pratiquement exempt d'urokinase à faible masse moléculaire
   d) il a un rapport activité fibrinolytique/activité estérasique supérieur à 2.000
   e) il est pratiquement exempt de contaminants thromboplastiniques (activité de coagulant zéro à la concentration de 200 UI/ml).

7. Immuno-adsorbant selon la revendication 4 ou 5, caractérisé en outre en ce que, quand il est utilisé pour la purification d'un précurseur d'urokinase humaine à chaîne unique, il donne un précurseur d'urokinase humaine ayant les caractéristiques suivantes:
   a) il s'agit d'une protéine à chaîne unique de masse moléculaire 50.000—54.000
   b) il est presque exempt d'activité amidolytique
   c) il a une forte activité de fibrine
   d) il n'est pas inactivé par les inhibiteurs du plasma
   e) il est converti en un activateur du plasminogène actif à deux chaînes par traitement à la plasmine.

8. Procédé pour la purification d'urokinase de masse moléculaire 54.000 ou de son précurseur d'urokinase à chaîne unique, à partir de sources biologiques, qui consiste à mettre en contact une source biologique ou un concentré de telles sources avec un immuno-adsorbant selon la revendication 4 ou 5, à un pH de 4,5 à 8,0, pour fixer sélectivement l'urokinase ayant une masse moléculaire de 54.000 ou son

EP 0 192 675 B1

précurseur d'urokinase à chaîne unique à l'immuno-adsorbant, à rincer avec une solution tamponnée à pH 6—8 et à libérer l'antigène fixé de l'immuno-adsorbant par élution avec une solution d'éluant, contenant facultativement du chlorure de sodium en solution aqueuse 0,5—1 M à un pH de 3,0 à 4,0.

9. Procédé selon la revendication 8, dans lequel la fixation de l'antigène à l'antigène à l'immuno-adsorbant peut aussi être obtenue en présence d'une solution aqueuse de chlorure de sodium à des concentrations allant jusqu'à 0,5 M.

10. Procédé selon la revendication 9, caractérisé en outre en ce qu'on obtient un produit urokinase qui a les caractéristiques suivantes:

a) il a un titre fibrinolytrique supérieur à 130.000 UI/ml

b) il est riche en urokinase à masse moléculaire élevée ou en son précurseur d'urokinase à chaîne unique

c) il est pratiquement exempt d'urokinase à faible mass moléculaire

d) il a un rapport activité fibrinolytique/activité estérasique supérieur à 2.000

e) il est pratiquement exempt de contaminants thromboplastiniques (activité de coagulant zéro à la concentration de 200 UI/ml).

11. Utilisation d'un anticorps monoclonal selon la revendication 1 ou 2, pour la purification de l'urokinase humaine ou de son précurseur d'urokinase à chaîne unique, par chromatographie d'affinité.

12. Utilisation d'un immuno-adsorbant selon l revendication 4 ou 5, pour la purification de l'urokinase humaine ou de son précurseur d'urokinase à chaîne unique par chromatographie d'affinité.

13. Procédé pour la préparation d'hybridomes produisant un anticorps monoclonal anti-urokinase capables, après culture, de produire un anticorps anti-urokinase selon la revendication 1, qui consiste à réaliser une fusion somatique de cellules d'un hôte immunisé avec une urokinase de masse moléculaire 54.000 avec des cellules de myélome de la même espèce animale, en présence d'un promoteur de fusion, et à trier les hybridomes producteurs souhaités au moyen d'un analyse enzymo-immunologique faisant appel à un anti-sérum anti-urokinase classique et aux dosages de la spécificité de classe, de la constante d'affinité et des épitopes spécifiques de l'anticorps produit.

14. Procédé selon la revendication 13, dans lequel les cellules somatiques sont des cellules spléniques de souris et les cellules de myélome sont des cellules de myélome de souris.

15. Procédé selon la revendication 13, dans lequel les cellules de myélome sont des cellules de myélome de souris connues sous la désignation X63-Ag8653.

16. Procédé selon la revendication 13, dans lequel l'hôte immunisé avec l'urokinase est un lapin.

17. Procédé selon la revendication 13, dans lequel le promoteur de fusion est un polyéthylèneglycol.

18. Procédé selon la revendication 13, dans lequel le promoteur de fusion est le polyéthylèneglycol 6000 (PEG 6000).

19. Procédé pour la préparation d'un anticorps monoclonal anti-urokinase selon la revendication 1, qui consiste à cultiver dans un hôte approprié ou dans un milieu de culture un hybridome obtenu selon la revendication 13.

20. Méthode sandwich à double anticorps pour détecter ou doser un antigène choisi parmi l'urokinase humaine et l'un de ses précurseurs, qui consiste

a) à faire réagir une solution à l'essai, contenant l'antigène, avec un premier anticorps anti-urokinase immobilisé sur un support en phase solide pour fixer l'antigène à la phase solide,

b) à mettre en contact avec l'antigène fixé une solution contenant un deuxième anticorps anti-urokinase auquel est lié un marqueur déterminable, l'un desdits premier et deuxième anticorps étant un anticorps monoclonal selon la revendication 1 ou 2

c) à détecter ou doser l'antigène en fonction de la mesure dans laquelle le marqueur déterminable a été fixé au support en phase solide.

21. Procédé selon la revendication 20, dans lequel le ''premier'' anticorps est choisi parmi un anti-sérum anti-urokinase, une immunoglobuline G (IgGs) purifiée anti-urokinase et un anticorps monoclonal dont on sait qu'il se fixe à un site de l'antigène qui est différent du site de fixation du ''deuxième'' anticorps monoclonal, et le deuxième anticorps est un anticorps monoclonal selon la revendication 1 ou 2.

22. Procédé selon la revendication 20, dans lequel la solution à l'essai est choisie parmi les fluides biologiques dilués, non dilués ou concentrés, et les bouillons ou extraits de fermentation de micro-organismes ou cultures cellulaires obtenus par génie génétique.

23. Procédé selon la revendication 20, dans lequel le marqueur déterminable lié au ''deuxième'' anticorps est une enzyme.

24. Procédé selon la revendication 20, dans lequel le marqueur déterminable lié au ''deuxième'' anticorps est la raifort-peroxydase.

25. Procédé selon la revendication 20, dans lequel le support en phase solide est choisi parmi les particules de cellulose, de polyacrylamide, de dextrane réticulé, de caoutchouc siliconé, de verre microcristallin et de matières plastiques.

26. Procédé selon la revendication 20, dans lequel le support en phase solide est un matériau en matière plastique préformé, comme un tube, un disque ou une microplaque de polystyrène, de polypropylène ou polyvinylique.

27. Procédé selon la revendication 20, dans lequel le support en phase solide est une microplaque polyvinylique préformée.

21

28. Coffret pour test biologique, pour doser l'urokinase humaine (HMW) ou l'un de ses précurseurs à chaîne unique, qui comprend:

a) un support en phase solide sur la surface duquel est immobilisé un premier anticorps anti-urokinase, et

b) une solution ou une préparation lyophilisée d'un deuxième anticorps anti-urokinase auquel es fixé un marqueur déterminable, où l'un desdits premier et deuxième anticorps est un anticorps monoclonal selon la revendication 1.

29. Coffret selon la revendication 28, dans lequel ledit deuxième anticorps est un anticorps monoclonal selon la revendication 1.

30. Coffret selon la revendication 28, dans lequel ledit deuxième anticorps est un anticorps monoclonal selon la revendication 1, et ledit premier anticorps est choisi parmi un antisérum anti-urokinase, une ou plusieurs IgG purifiées anti-urokinase et un anticorps monoclonal qui fixe un site antigénique différent du site de fixation dudit deuxième anticorps.

31. Coffret selon la revendication 28, dans lequel l'un desdits premier et deuxième anticorps est un anticorps monoclonal selon la revendication 1, et l'autre est un anticorps monoclonal connu pour se fixer à un site différent de l'antigène.

32. Coffret selon la revendication 28, dans lequel ledit marqueur déterminable est une enzyme.

33. Coffret selon la revendication 28, dans lequel ledit marqueur déterminable est la raifort-peroxydase.

**Revendications pour l'Etat contractant: AT**

1. Procédé pour la préparation d'hybridomes produisant un anticorps monoclonal anti-urokinase capables, après culture, de produire un anticorps anti-urokinase ayant les caractéristiques suivantes:

a) il s'agit d'une IgG$_1$

b) il a une constante d'affinité de $(1,42 \pm 1,5) \times 10^7$ 1 mole$^{-1}$ pour l'urokinase humaine immobilisée, la mesure étant effectuée essentiellement par le mode opératoire décrit par Tsapis et al. dans Eur. J. Biochem. *64*, 369 (1976)

c) il fixe l'urokinase à masse moléculaire élevée (54.000) et/ou le précurseur d'urokinase à chaîne unique sans fixation de l'urokinase à faible masse moléculaire (33.000)

d) il fixe le fragment protéolytique 18.000 de la chaîne A de l'urokinase

e) il ne subit pas de réaction croisée avec les endopeptidases enzymatiquement actives sur le sérum ou l'urine autres que l'urokinase,

qui consiste à réaliser une fusion somatique de cellules d'un hôte immunisé avec une urokinase de masse moléculaire 54.000 avec des cellules de myélome de la même espèce animale, en présence d'un promoteur de fusion, et à trier les hybridomes producteurs souhaités au moyen d'une analyse enzymo-immunologique faisant appel à un anti-sérum anti-urokinase classique et aux dosages de la spécificité de classe, de la constante d'affinité et des épitopes spécifiques de l'anticorps produit.

2. Procédé selon la revendication 1, dans lequel les cellules somatiques sont des cellules spléniques de souris et les cellules de myélome son des cellules de myélome de souris.

3. Procédé selon la revendication 1, dans lequel les cellules de myélome sont des cellules de myélome de souris connues sous la désignation X63-Ag8653.

4. Procédé selon la revendication 1, dans lequel l'hôte immunisé avec l'urokinase est un lapin.

5. Procédé selon la revendication 1, dans lequel le promoteur de fusion est un polyéthylèneglycol.

6. Procédé selon la revendication 5, dans lequel le promoteur de fusion est le polyéthylèneglycol 6000 (PEG 6000).

7. Procédé pour la préparation d'un anticorps monoclonal anti-urokinase selon la revendication 1, qui consiste à cultiver dans un hôte approprié ou dans un milieu de culture un hybridome obtenu selon la revendication 1.

8. Procédé de préparation d'un anticorps monoclonal anti-urokinase humaine selon la revendication 1, caractérisé en outre en ce que

a) il ne perturbe pas l'activité enzymatrique des l'urokinase

b) il a un point isoélectrique d'environ 5,75.

9. Procédé selon la revendication 1 de préparation d'un anticorps monoclonal anti-urokinase humaine, caractérisé en outre en ce que, quand il est couplé à une matrice appropriée, il est capable de fixer l'antigène à un pH de 4,5 à 8,0 même en présence de chlorure de sodium aqueux 0,5 M, et de libérer l'anticorps fixé à un pH de 3,0 à 4,0 dans du chlorure de sodium aqueux 0,5—1 M.

10. Procédé pour la purification d'urokinase de masse moléculaire 54.000 ou de son précurseur d'urokinase à chaîne unique, à partir de sources biologiques, qui consiste à mettre en contact une source biologique ou un concentré telles sources avec un immuno-adsorbant que l'on obtient en liant un anticorps anti-urokinase ayant les caractéristiques de l'anticorps monoclonal de la revendication 1 à une matrice choisie parmi l'agarose, l'agarose modifiée et activée, le dextrane réticulé, la cellulose et la carboxyméthylcellulose, à un pH de 4,5 à 8,0 pour fixer sélectivement l'urokinase ayant une masse moléculaire de 54.000 ou son précurseur d'urokinase à chaîne unique à l'immuno-adsorbant, à rincer avec une solution tamponnée à pH 6—8 et à libérer l'antigène fixé de l'immuno-adsorbant par élution avec une solution d'éluant, contenant facultativement du chlorure de sodium en solution aqueuse 0,5—1 M à un pH de 3,0 à 4,0.

11. Procédé selon la revendication 10, dans lequel la fixation de l'antigène à l'immuno-adsorbant peut aussi être obtenue en présence d'une solution aqueuse de chlorure de sodium à des concentrations allant jusqu'à 0,5 M.

12. Procédé selon la revendication 10, caractérisé en outre en ce qu'on obtient un produit urokinase qui a les caractéristiques suivantes:

a) il a un titre fibrinolytique supérieur à 130.000 UI/ml

b) il est riche en urokinase à masse moléculaire élevée ou en son précurseur d'urokinase à chaîne unique

c) il est pratiquement exempt d'urokinase à faible masse moléculaire

d) il a un rapport activité fibrinolytique/activité estérasique supérieur à 2.000

e) il est pratiquement exempt de contaminants thromboplastiniques (activité de coagulant zéro à la concentration de 200 UI/ml).

13. Procédé selon la revendication 10, caractérisé en outre en ce qu'on obtient un précurseur d'urokinase humaine ayant les caractéristiques suivantes:

a) il s'agit d'une protéine à chaîne unique de masse moléculaire 50.000—54.000

b) il est presque exempt d'activité amidolytique

c) il a une forte activité de fibrine

d) il n'est pas inactivé par les inhibiteurs du plasma

e) il est converti en un activateur du plasminogène actif à deux chaînes par traitement à la plasmine.

14. Procédé selon la revendication 10 dans lequel l'immuno-adsorbant pour la purification de l'urokinase humaine et/ou de son précurseur d'urokinase à chaîne unique, est obtenu en liant l'anticorps monoclonal défini à la revendication 1 à une matrice constituée d'agarose.

15. Procédé selon la revendication 10, dans lequel l'anticorps monoclonal est caractérisé en outre en ce que

a) il ne perturbe pas l'activité enzymatique de l'urokinase

b) il a un point isoélectrique d'environ 5,75.

16. Méthode sandwich à double anticorps pour détecter ou doser un antigène choisi parmi l'urokinase humaine et l'un de ses précurseurs, qui consiste

a) à faire réagir une solution à l'essai, contenant l'antigène, avec un premier anticorps anti-urokinase immobilisé sur un support en phase solide pour fixer l'antigène à la phase solide,

b) à mettre en contact avec l'antigène fixé une solution contenant un deuxième anticorps anti-urokinase auquel est lié un marqueur déterminable, l'un desdits premier et deuxième anticorps étant un anticorps monoclonal ayant les caractéristiques de l'anticorps monoclonal la revendication 1,

c) à détecter ou doser l'antigène en fonction de la mesure dans laquelle le marqueur déterminable a été fixé au support en phase solide.

17. Procédé selon la revendication 1, dans lequel ledit anticorps monoclonal est caractérisé en outre en ce que

a) il ne perturbe pas l'activité enzymatique de l'urokinase

b) il a un point isoélectrique d'environ 5,75.

18. Procédé selon la revendication 1, dans lequel le "premier" anticorps est choisi parmi un anti-sérum anti-urokinase, une immunoglobuline G purifiée anti-urokinase et un anticorps monoclonal dont on sait qu'il se fixe à un site de l'antigène qui est différent du site de fixation du "deuxième" anticorps monoclonal, et le deuxième anticorps est un anticorps monoclonal selon la revendication 1 ou 2.

19. Procédé selon la revendication 1, dans lequel la solution à l'essai est choisie parmi les fluides biologiques dilués, non dilués ou concentrés, et les bouillons ou extraits de fermentation de micro-organismes ou cultures cellulaires obtenus par génie génétique.

20. Procédé selon la revendication 1, dans lequel le marqueur déterminable lié au "deuxième" anticorps est une enzyme.

21. Procédé selon la revendication 1, dans lequel le marqueur déterminable lié au "deuxième" anticorps est la raifort-peroxydase.

22. Procédé selon la revendication 1, dans lequel le support en phase solide est choisi parmi les particules de cellulose, de polyacrylamide, de dextrane réticulé, de caoutchouc siliconé, de verre microcristallin et de matières plastiques.

23. Procédé selon la revendication 1, dans lequel le support en phase solide est un matériau en matière plastique préformé, comme un tube, un disque ou une microplaque de polystyrène, de polypropylène ou polyvinylique.

24. Procédé selon la revendication 1, dans lequel le support en phase solide est une microplaque polyvinylique préformée.